# FASCICULE DE BREVET EUROPEEN

(11) **EP 2 158 201 B1**
(45) Date de publication et mention de la délivrance du brevet: **13.04.2011**
(21) Numéro de dépôt: 08826268.8
(22) Date de dépôt: 11.06.2008
(51) Int. Cl.: C07D 471/04

(54) **DERIVES DE 7-ALKYNYL-1,8-NAPHTHYRIDONES, LEUR PREPARATION ET LEUR APPLICATION EN THERAPEUTIQUE**
7-ALKYNYL-1,8-NAPHTHYRIDON-DERIVATE, VERFAHREN ZU IHRER HERSTELLUNG UND IHRE VERWENDUNG FÜR THERAPEUTIKA
DERIVATIVES OF 7-ALKYNYL-1,8-NAPHTHYRIDONES, PREPARATION METHOD THEREOF AND USE OF SAME IN THERAPEUTICS

(30) Priorité: 13.06.2007 FR 0704192
(43) Date de publication de la demande: 03.03.2010
(73) Titulaire: Sanofi-Aventis, 75013 Paris (FR)
(72) Inventeur: ALAM, Antoine, F-75013 Paris (FR); BISCARRAT, Sandrine, F-75013 Paris (FR); BLANC, Isabelle, F-75013 Paris (FR); BONO, Françoise, F-75013 Paris (FR); DUCLOS, Olivier, F-75013 Paris (FR); MC CORT, Gary, F-75013 Paris (FR)
(74) Mandataire: Romanowski, Caroline
(86) Numéro de dépôt international: PCT/FR2008/000793
(87) Numéro de publication internationale: WO 2009/007535

(56) Documents cités:
- EP-A- 0 978 516
- WO-A-97/04775
- WO-A-02/094823
- DATABASE CA [Online] CHEMICAL ABSTRACTS SERVICE, COLUMBUS, OHIO, US; NAGASAKI, SENKICHI ET AL: "Antibacterial activity of 1-substituted 1,4-dihydro-7-[2-(5-nitro-2- furyl)vinyl]-4-oxo-1,8-naphthyridine derivatives" XP002463821 extrait de STN Database accession no. 1972:456445 & CHEMICAL & PHARMACEUTICAL BULLETIN , 20(4), 639-49 CODEN: CPBTAL; ISSN: 0009-2363, 1972,

## Description

La présente invention se rapporte à des dérivés de 7-alkynyl-1,8-naphtyridones, à leur préparation et à leur application en thérapeutique.

La présente invention a pour objet des composés répondant à la formule (1) : 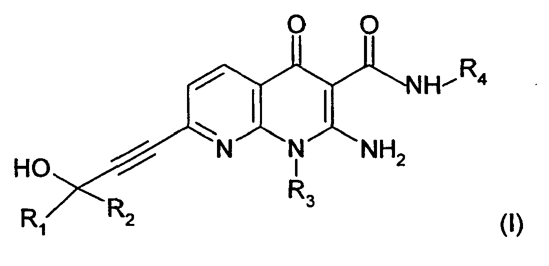 dans laquelle :
R₁ et R₂
   **(1)** représentent indépendamment l'un de l'autre:
      soit un atome d'hydrogène,
      soit un groupe alkyle en C1-C7, un groupe -CO-alkyle en C1-C7 ou un groupe cycloalkyle en C3-C8, où lesdits groupes alkyle et cycloalkyle sont éventuellement substitués par un ou plusieurs groupes choisis parmi les atomes d'halogène et les groupes hydroxy et alcoxy,
      soit un groupe phényle éventuellement substitué par un ou plusieurs groupes choisis parmi les atomes d'halogène, les groupes alkyles en C1-C4, alcoxy en C1-C4, hydroxy, -halogénoalcoxy, -halogénoalkyle, -CN, -NRR', où R et R' sont tels que définis ci-dessous,
      soit un groupe hétéroaryle éventuellement substitué en des positions quelconques, y compris sur un atome d'azote dudit hétéroaryle, par un ou plusieurs groupes choisis parmi les atomes d'halogène, les groupes alkyles en C1-C4 et les groupes -NRR' où R et R' sont tels que définis ci-dessous,
   **(2)** ou R₁ et R₂ forment ensemble, avec l'atome de carbone qui les porte:
      soit un groupe cycloalkyle en C4-C8 ;
      soit un groupe hétérocyclique saturé de 4 à 8 chaînons comprenant un hétéroatome choisi parmi les atomes N, O et S,
      et ledit groupe hétérocyclique pouvant être fusionné avec un groupe phényle ;
R₃ représente :
   soit un groupe alkyle en C1-C7 linéaire ou ramifié ou un groupe alkyle en C3-C7 dont au moins 3 atomes de carbone sont cyclisée, ledit groupe alkyle étant éventuellement substitué par un ou plusieurs groupes choisis parmi les atomes d'halogène et les groupes hydroxy, alcoxy, -NRR', -halogénoalkyle et -SO₂-(C1-C4)alkyl, où R et R' sont tels que définis ci-après ;
   soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O et S, où ledit groupe hétérocyclique est éventuellement substitué par un groupe oxo,
   soit un groupe -(CH₂)-hétéroaryle, où n = 0, 1, 2 ou 3 et où le groupe hétéroaryle comprend 5 ou 6 chaînons et comprend un ou plusieurs hétéroatomes choisis parmi l'azote, l'oxygène et le soufre ;
R₄ représente un atome d'hydrogène, un groupe alkyle en C1-C4 ou un groupe alcoxy en C1-C4 ;
R et R' représentent indépendamment l'un de l'autre un atome d'hydrogène, un groupe alkyle en C1-C4.
WO 97/04776 , EP 0 978 516 et WO 02/094823 décrivent des composés, inhibiteurs de PDE IV et/ou de TNF-alpha, pouvant comporter un noyau naphtyridone dont les substitutions, respectivement en positions (i) 2, 3 et 7, (ii) 2 et 7 et (iii) 1, 2 et 7, sont différentes de celles prévues pour les dérivés naphtyridones de formule (1) selon la présente invention.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leur mélange y compris leur mélange racémique font partie de l'invention.

Les composés de formule (1) peuvent exister à l'état de bases ou salifiés par des acides ou des bases, notamment des acides ou des bases pharmaceutiquement acceptables. De tels sels d'addition font partie de l'invention. Ces sels sont avantageusement préparés avec des acides ou des bases pharmaceutiquement acceptables, mais les sels d'autres acides ou de bases utiles, par exemple, pour la purification ou l'isolement des composés de formule (I), font également partie de l'invention.

Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, et sauf mention différente dans le texte, on entend par :
- un groupe alkyle : un groupe aliphatique saturé comprenant de 1 à 7 atomes de carbone (avantageusement, de 1 à 4 atomes de carbone) et étant linéaire ou, quand la chaîne alkyle comprend au moins 3 atomes de carbone, pouvant être linéaire, ramifié ou partiellement cyclisé. A titre d'exemples, on peut citer les groupes méthyle, éthyle, propyle, isopropyle, butyle, iso-butyle, tertio-butyle, méthylène-cyclopropyle, méthylène-cyclohexyle, pentyle, 2,2-diméthylpropyle, hexyle, heptyle, etc ;
- un groupe cycloalkyle : un groupe alkyle cyclique comprenant de 3 à 8 atomes de carbone et dont tous les atomes de carbones sont engagés dans le cycle. On peut citer les groupes cyclopropyle, cyclobutyle, cyclopentyle, cyclohexyle et cycloheptyle ;
- un groupe alcoxy : un groupe -O-alkyle, où le groupe alkyle est tel que défini ci-dessus ;
- un atome d'halogène : un fluor, un chlore, un brome ou un iode ;
- un groupe halogénoalkyle : un groupe alkyle dont un ou plusieurs atomes d'hydrogène ont été substitués par un atome d'halogène ; à titre d'exemple, on peut citer -CF₃ ;
- un groupe halogénoalcoxy : un groupe alcoxy dont un ou plusieurs atomes d'hydrogène ont été substitués par un atome d'halogène ; à titre d'exemple, on peut citer -OCF₃ ;
- un groupe aryle : un groupe aromatique monocyclique, tel qu'un groupe phényle ;
- un groupe hétéroaryle : un groupe aromatique comprenant 5 ou 6 chaînons et comprenant un ou plusieurs hétéroatomes choisis parmi l'azote, l'oxygène et le soufre. On peut citer par exemple les groupes pyridinyle, furanyle, thiényle, pyrimidyle, pyrazinyle et thiazolyle ; et
- un groupe hétérocyclique : un groupe alkyle cyclique comprenant entre 4 et 8 chaînons et comprenant un ou plusieurs hétéroatomes choisis parmi l'azote, l'oxygène et le soufre. On peut citer par exemple les groupes pipéridinyle, pyrrolidinyle, oxétanyle, tétrahydrofuranyle, tétrahydropyranyle et tétrahydrothiényle.

Parmi les composés de formule (I) objets de l'invention, on peut citer un sous-groupe de composés qui se définit comme suit :
R₁ et R₂
   **(3)** représentent indépendamment l'un de l'autre:
      soit un atome d'hydrogène,
      soit un groupe alkyle en C1-C7, un groupe -CO-alkyle en C1-C7 ou un groupe cycloalkyle en C3-C8, où lesdits groupes alkyle et cycloalkyle sont éventuellement substitués par un ou plusieurs groupes choisis parmi les atomes d'halogène et les groupes hydroxy et alcoxy,
      soit un groupe phényle éventuellement substitué par un ou plusieurs groupes choisis parmi les atomes d'halogène, les groupes alkyles en C1-C4, alcoxy en C1-C4, hydroxy, -OCF₃, -CF₃, -CN, -NRR', où R et R' sont tels que définis ci-dessous,
      soit un groupe hétéroaryle éventuellement substitué en des positions quelconques, y compris sur un atome d'azote dudit hétéroaryle, par un ou plusieurs groupes choisis parmi les atomes d'halogène, les groupes alkyles en C1-C4 et les groupes -NRR' où R et R' sont tels que définis ci-dessous,
   **(4)** ou R₁ et R₂ forment ensemble, avec l'atome de carbone qui les porte:
      soit un groupe cycloalkyle en C4-C8 ;
      soit un groupe hétérocyclique saturé de 4 à 8 chaînons comprenant un hétéroatome choisi parmi les atomes N, O et S,
         et ledit groupe hétérocyclique pouvant être fusionné avec un groupe phényle ;
R₃ représente :
   soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les atomes d'halogène et les groupes hydroxy, alcoxy, -NRR', -CF₃ et -SO₂-(C1-C4)alkyl, où R et R' sont tels que définis ci-après ;
   soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O et S, où ledit groupe hétérocyclique est éventuellement substitué par un groupe oxo,
   soit un groupe -(CH₂)ₙ-hétéroaryle, où n = 0, 1, 2 ou 3 et où le groupe hétéroaryle comprend 5 ou 6 chaînons et comprend un ou plusieurs hétéroatomes choisis parmi l'azote, l'oxygène et le soufre :
R₄ représente un atome d'hydrogène, un groupe alkyle en C1-C4 ou un groupe alcoxy en C1-C4 ;
R et R' représentent indépendamment l'un de l'autre un atome d'hydrogène, un groupe alkyle en C1-C4.

Parmi les composés de formule (I) objets de l'invention, on peut citer un sous-groupe de composés qui se définit comme suit :
R₁ et R₂
**(1)** représentent indépendamment l'un de l'autre:
   - soit un atome d'hydrogène,
   - soit un groupe alkyle en C1-C7, un groupe -CO-alkyle en C1-C7 ou un groupe cycloalkyle en C3-C8, où ledit groupe alkyle est éventuellement substitué par un ou plusieurs groupes choisis parmi les groupes hydroxy et alcoxy,
   - soit un groupe phényle éventuellement substitué par un ou plusieurs groupes alcoxy en C1-C4,
   - soit un groupe hétéroaryle,
**(2)** ou R₁ et R₂ forment ensemble, avec l'atome de carbone qui les porte:
   - soit un groupe cycloalkyle en C4-C8 ;
   - soit un groupe hétérocyclique saturé de 4 à 8 chaînons comprenant un hétéroatome choisi parmi les atomes N, O et S,
   et ledit groupe hétérocyclique pouvant être fusionné avec un groupe phényle ; et/ou
R₃ représente :
   - soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, -NRR', halogénoalkyle et -SO₂-(C1-C4)alkyl, où R et R' sont tels que définis ci-après ;
   - soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N et O où ledit groupe hétérocyclique est éventuellement substitué par un groupe oxo,
   - soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
   et/ou
R₄ représente un groupe alkyle en C1-C4 ou un groupe alcoxy en C1-C4 ;
   et/ou
R et R' représentent chacun un groupe alkyle en C1-C4 linéaire ou ramifié.

Parmi les composés de formule (I) objets de l'invention, on peut citer un autre sous-groupe de composés qui se définit comme suit :
R₁ et R₂
   **(1)** représentent indépendamment l'un de l'autre:
      - soit un atome d'hydrogène,
      - soit un groupe alkyle en C1-C7, un groupe -CO-alkyle en C1-C7 ou un groupe cycloalkyle en C3-C8, où ledit groupe alkyle est éventuellement substitué par un ou plusieurs groupes choisis parmi les groupes hydroxy et alcoxy,
      - soit un groupe phényle éventuellement substitué par un ou plusieurs groupes alcoxy en C1-C4, ou
      - soit un groupe hétéroaryle,
   **(2)** ou R₁ et R₂ forment ensemble, avec l'atome de carbone qui les porte:
      - soit un groupe cycloalkyle en C4-C8 ;
      - soit un groupe hétérocyclique saturé de 4 à 8 chaînons comprenant un hétéroatome d'oxygène,
   et ledit groupe hétérocyclique pouvant être fusionné avec un groupe phényle ;
   et/ou
R₃ représente :
   - soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, -NRR', halogénoalkyle et -SO₂-(C1-C4)alkyl, où R et R' sont tels que définis ci-après ;
   - soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O, où ledit groupe hétérocyclique est éventuellement substitué par un groupe oxo,
   - soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
   et/ou
R₄ représente un groupe alkyle en C1-C4 ou un groupe alcoxy en C1-C4 ;
   et/ou
R et R' représentent chacun un groupe alkyle en C1-C4 linéaire ou ramifié.

Parmi les composés de formule (I) objets de l'invention, on peut citer un troisième sous-groupe de composés qui se définit comme suit :
B₁ et R₂
   (1) représentent indépendamment l'un de l'autre:
      - soit un atome d'hydrogène,
      - soit un groupe alkyle en C1-C7, ou un groupe cycloalkyle en C3-C8, où ledit groupe alkyle est éventuellement substitué par un ou plusieurs groupes choisis parmi les groupes hydroxy et alcoxy,
      - soit un groupe phényle éventuellement substitué par un ou plusieurs groupes alcoxy en C1-C4,
      - soit un groupe hétéroaryle,
   (2) ou R₁ et R₂ forment ensemble, avec l'atome de carbone qui les porte un groupe cycloalkyle en C4-C8 ;
   et/ou
R₃ représente :
   - soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, halogénoalkyle et -SO₂-(C1-C4)alkyl ;
   - soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O, où ledit groupe hétérocyclique est éventuellement substitué par un groupe oxo,
   - soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
   et/ou
R₄ représente un groupe alkyle en C1-C4;
   et/ou
R et R' représentent chacun un groupe alkyle en C1-C4 linéaire ou ramifié.

Parmi les composés de formule (I) objets de l'invention, on peut citer un sous-groupe de composés pour lesquels R₁ et R₂
(1) représentent indépendamment l'un de l'autre:
   - soit un atome d'hydrogène,
   - soit un groupe alkyle en C1-C7, un groupe -CO-alkyle en C1-C7 ou un groupe cycloalkyle en C3-C8, où ledit groupe alkyle est éventuellement substitué par un ou plusieurs groupes choisis parmi les groupes hydroxy et alcoxy,
   - soit un groupe phényle éventuellement substitué par un ou plusieurs groupes alcoxy en C1-C4,
   - soit un groupe hétéroaryle,
(2) ou R₁ et R₂ forment ensemble, avec l'atome de carbone qui les porte:
   - soit un groupe cycloalkyle en C4-C8 ;
   - soit un groupe hétérocyclique saturé de 4 à 8 chaînons comprenant un hétéroatome choisi parmi les atomes N, O et S,
et ledit groupe hétérocyclique pouvant être fusionné avec un groupe phényle ;

Plus particulièrement, parmi les composés de formule (I) objets de l'invention, on peut citer un sous-groupe de composés pour lesquels R₁ et/ou R₂ représentent un groupe pyridinyle, thiényle, thiazolyle ou pyrazinyle ou imidazolyle.

Plus particulièrement, parmi les composés de formule (I) objets de l'invention, on peut citer un sous-groupe de composés pour lesquels R₁ et R₂ forment ensemble, avec l'atome de carbone qui les porte un hétérocyclique choisi parmi un groupe tétrahydrofuranyle ou tétrahydropyranyle, ledit groupe hétérocyclique pouvant être fusionné avec un groupe phényle.

Parmi les composés de formule (I) objets de l'invention, on peut citer un sous-groupe de composés pour lesquels R₃ représente :
- soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, -NRR', halogénoalkyle et -SO₂-(C1-C4)alkyl, où R et R' sont tels que définis ci-après ;
- soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N et O, où ledit groupe hétérocyclique est éventuellement substitué par un groupe oxo,
- soit un groupe -(CH₂)ₙ-hétéroaryle, où n = 0 ou 1 et où le groupe hétéroaryle comprend 5 ou 6 chaînons et comprend un ou plusieurs hétéroatomes d'azote.

Plus particulièrement, parmi les composés de formule (I) objets de l'invention, on peut citer un sous-groupe de composés pour lesquels R₃ représente un groupe alkyle en C3-C7 dont au moins 3 atomes de carbone sont cyclisés choisi parmi les groupes cyclopropyle, cyclobutyle, cyclopentyle, cyclohexyle et méthylène-cyclopropyle.

Plus particulièrement, parmi les composés de formule (I) objets de l'invention, on peut citer un sous-groupe de composés pour lesquels R₃ représente un groupe -(CH₂)ₙ hétérocyclique, où n = 0, 1, 2 ou 3 et où le groupe hétérocyclique est choisi parmi les groupes tétrahydropyranyle, tétrahydrofuranyle ou pyrrolidinyle.

Plus particulièrement, parmi les composés de formule (I) objets de l'invention, on peut citer un sous-groupe de composés pour lesquels R₃ représente un groupe - (CH₂)ₙ-hétéroaryle, où n = 1 et où le groupe hétéroaryle représente un groupe pyridinyle.

Parmi les composés de formule (I) objets de l'invention, on peut citer un sous-groupe de composés pour lesquels R₄ représente un groupe alkyle en C1-C4 ou un groupe alcoxy en C1-C4 ;

Parmi les composés de formule (I) objets de l'invention, on peut citer un sous-groupe de composés pour lesquels R et R' représentent chacun un groupe alkyle en C1-C4 linéaire ou ramifié.

Parmi les composés objets de l'invention, on peut notamment citer les composés suivants :
■ 2-amino-1-éthyl-7-(3-hydroxy-3-méthylbut-1-yn-1-yl)-N-méthyl-4-oxo-1,4-dihydro-1,8-naphthyridine-3-carboxamide
■ (±)-2-amino-1-éthyl-7-[(1-hydroxycyclopentyl)éthynyl]-N-méthyl-4-oxo-1,4-dihydro-1,8-naphthyridine-3-carboxamide
■ (±)-2-amino-1-éthyl-7-(3-hydroxybut-1-yn-1-yl)-N-méthyl-4-oxo-1,4-dihydro-1,8-naphthyridine-3-carboxamide
■ (±)-2-amino-1-éthyl-7-(3-hydroxy-3-méthylpent-1-yn-1-yl)-N-méthyl-4-oxo-1,4-dihydro-1,8-naphthyridine-3-carboxamide
■ (±)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-7-(3-cyclopentyl-3-hydroxyprop-1-yn-1-yl)-1-éthyl-N-méthyl-4-oxo-1,4-dihydro-1,8-naphthyridine-3-carboxamide
■ (±)-2-amino-7-(3-cyclopropyl-3-hydroxyprop-1-yn-1-yl)-1-éthyl-N-méthyl-4-oxo-1,4-dihydro-1,8-naphthyridine-3-carboxamide
■ 2-amino-1-éthyl-7-[(1-hydroxycyclobutyl)éthynyl]-N-méthyl-4-oxo-1,4-dihydro-1,8-naphthyridine-3-carboxamide
■ (±)-2-amino-7-(3,4-dihydroxy-3-méthylbul-1-yn-1-yl)-1-éthyl-N-méthyl-4-oxo-1,4-dihydro-1,8-naphthyridine-3-carboxamide
■ 2-amino-1-éthyl-7-[3-hydroxy-4-méthoxy-3-(méthoxyméthyl)but-1-yn-1-yl]-N-méthyl-4-oxo-1,4-dihydro-1,8-naphthyridine-3-carboxamide
■ (±)-2-amino-1-(cyclopropylméthyl)-7-(3-hydroxy-4-methoxy-3-méthylbut-1-yn-1-yl)-N-méthyl-4-oxo-1,4-dihydro-1,8-naphthyridine-3-carboxamide
■ (±)-2-amino-1-éthyl-7-(3-hydroxy-3-phénylbut-1-yn-1-yl)-N-méthyl-4-oxo-1,4-dihydro-1,8-naphthyridine-3-carboxamide
■ (±)-2-amino-1-éthyl-7-[3-hydroxy-3-(3-thiényl)but-1-yn-1-yl]-N-méthyl-4-oxo-1,4-dihydro-1,8-naphthyridine-3-carboxamide
■ (±)-2-amino-1-éthyl-7-[3-hydroxy-3-(3-méthoxyphényl)but-1-yn-1-yl]-N-méthyl-4-oxo-1,4-dihydro-1,8-naphthyridine-3-carboxamide
■ (±)-2-amino-1-éthyl-7-[3-hydroxy-3-(4-méthoxyphényl)but-1-yn-1-yl]-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-1-(3-méthoxypropyl)-N-méthyl-4-oxo-1,4-dihydro-1,8-naphthyridine-3-carboxamide
■ (±)-2-amino-1-cyclopentyl-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-1-isopropyl-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-1-isobutyl-N-méthyl-4-oxo-1,4-dihydro-1,8-naphthyridine-3-carboxamide
■ 2-amino-7-(3-hydroxy-3-méthylbut-1-yn-1-yl)-1-(3-méthoxypropyl)-N-méthyl-4-oxo-1,4-dihydro-1,8-naphthyridine-3-carboxamide
■ (±)-2-amino-1-éthyl-7-(3-hydroxy-4-méthoxy-3-phénylbut-1-yn-1-yl)-N-méthyl-4-oxo-1,4-dihydro-1,8-naphthyridine-3-carboxamide
■ (±)-2-amino-1-(tétrahydropyran-4-yl)-7-(3-hydroxy-4-méthoxy-3-méthylbut-1 - yn-1-yl)-N-méthyl-4-oxo-1,4-dihydro-1,8-naphthyridine-3-carboxamide
■ (±)-2-amino-1-cyclohexyl-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-1-éthyl-7-[3-hydroxy-3-(1,3-thiazo)-2-yl)but-1-yn-1-yl]-N-méthyl-4-oxo-1,4-dihydro-1,8-naphthyridine-3-carboxamide
■ (±)-2-amino-1-éthyl-7-[3-hydroxy-3-(méthoxyméthyl)pent-1-yn-1-yl]-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-[3-(2-oxopyrrolidin-1-yl)propyl]-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-propyl-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-(2,2,2-trifluoroéthyl)-1,4-dihydro-1,8-naphthyridine-3-carboxamide
■ (±)-2-amino-7-(3-hydroxy-3-méthylbut-1-yn-1-yl)-N-méthy)-4-oxo-1-(tetrahydrofuran-2-ylméthyl)-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-(pyridin-2-ylméthyl)-1,4-dihydro-1,8-naphthyridine-3-carboxamide
■ (±)-2-amino-1-éthyl-7-(3-hydroxy-3-pyrazin-2-ylbut-1-yn-1-yl)-N-méthyl-4-oxo-1,4-dihydro-1,8-naphthyridine-3-carboxamide
■ (±)-2-amino-7-(3-cyclopropyl-3-hydroxy-4-méthoxybut-1-yn-1-yl)-1-éthyl-N-méthyl-4-oxo-1,4-dihydro-1,8-naphthyridine-3-carboxamide
■ (±)-2-amino-1-éthyl-7-[3-hydroxy-3-(2-thiényl)but-1-yn-1-yl]-N-méthyl-4-oxo-1,4-dihydro-1,8-naphthyridine-3-carboxamide
■ (±)-2-amino-1-éthyl-7-[3-hydroxy-3-(2-méthoxyphényl)but-1-yn-1-yl]-N-méthyl-4-oxo-1,4-dihydro-,8-naphthyridine-3-carboxamide
■ (±)-2-amino-1-cyclopentyl-7-(3,4-dihydroxy-3-méthylbut-1-yn-1-yl)-N-méthyl-4-oxo-1,4-dihydro-1,8-naphthyridine-3-carboxamide
■ (±)-2-amino-7-(4-éthoxy-3-hydroxy-3-méthylbut-1-yn-1-yl)-1-éthyl-N-méthyl-4-oxo-1,4-dihydro-1,8-naphthyridine-3-carboxamide
■ (±)-2-amino-1-(trans-4-hydroxycyclohexyl)-7-(3-hydroxy-4-méthoxy-3-méthylbut-1-yn-1-yl)-N-méthyl-4-oxo-1,4-dihydro-1,8-naphthyridine-3-carboxamide (±)-2-amino-7-(3-hydroxy-4-méthoxy-3-méthylbut-1-yn-1-yl)-N-méthyl-1-[3-(méthylsulfonyl)propyl]-4-oxo-1,4-dihydro-1,8-naphthyridine-3-carboxamide
■ (-)-2-amino-1-(cyclopropylméthyl)-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-(cyclopropylméthyl)-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-7-(3,4-dihydroxy-3-méthylbut-1-yn-1-yl)-1-éthyl-N-méthyl-4-oxo-1,4-dihydro-1,8-naphthyridine-3-carboxamide
■ (-)-2-amino-7-(3,4-dihydroxy-3-méthylbut-1-yn-1-yl)-1-éthyl-N-méthyl-4-oxo-1,4-dihydro-1,8-naphthyridine-3-carboxamide
■ (-)-2-amino-1-éthyl-7-(3-hydroxy-3-phénylbut-1-yn-1-yl)-N-méthyl-4-oxo-1,4-dihydro-1,8-naphthyridine-3-carboxamide
■ (+)-2-amino-1-éthyl-7-(3-hydroxy-3-phénylbut-1-yn-1-yl)-N-méthyl-4-oxo-1,4-dihydro-1,8-naphthyridine-3-carboxamide
■ (-)-2-amino-7-(3-hydroxy-4-méthoxy-3-méthylbut-1-yn-1-yl)-1-(3-méthoxypropyl)-N-méthyl-4-oxo-1,4-dihydro-1,8-naphthyridine-3-carboxamide
■ (+)-2-amino-7-(3-hydroxy-4-méthoxy-3-méthylbut-1-yn-1-yl)-1-(3-méthoxypropyl)-N-méthyl-4-oxo-1,4-dihydro-1,8-naphthyridine-3-carboxamide
■ (-)-2-amino-1-cyclopentyl-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-cyclopentyl-7-(3-hydroxy-4-méthoxy-3-méthylbut-1-yn-1-yl)-N-méthyl-4-oxo-1,4-dihydro-1,8-naphthyridine-3-carboxamide
■ (-)-2-amino-7-(3-hydroxy-4-méthoxy-3-méthylbut-1-yn-1-yl)-1-isopropyl-N-méthyl-4-oxo-1,4-dihydro-1,8-naphthyridine-3-carboxamide
■ (+)-2-amino-7-(3-hydroxy-4-méthoxy-3-méthylbut-1-yn-1-yl)-1-isopropyl-N-méthyl-4-oxo-1,4-dihydro-1,8-naphthyridine-3-carboxamide
■ (-)-2-amino-1-cyclohexyl-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-cyclohexyl-7-(3-hydroxy-4-méthoxy-3-méthylbut-1-yn-1-yl)-N-méthyl-4-oxo-1,4-dihydro-1, 8-naphthyridine-3-carboxamide
■ (-)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide
■ (-)-2-amino-7-(3-hydroxy-4-méthoxy-3-méthyl but-1-yn-1-yl)-N-méthyl-4-oxo-1-propyl-1,4-dihydro-1,8-naphthyridine-3-carboxamide
■ (+)-2-amino-7-(3-hydroxy-4-méthoxy-3-méthylbut-1-yn-1-yl)-N-méthyl-4-oxo-1-propyl-1,4-dihydro-1,8-naphthyridine-3-carboxamide
■ (-)-2-amino-7-(3-hydroxy-4-méthoxy-3-méthylbut-1-yn-1-yl)-N-méthyl-4-oxo-1-(2,2,2-triftuoroéthyl)-1,4-dihydro-1,8-naphthyridine-3-carboxamide
■ (+)-2-amino-7-(3-hydroxy-4-méthoxy-3-méthylbut-1-yn-1-yl)-N-méthyl-4-oxo-1-(2,2,2-trifluoroéthyl)-1,4-dihydro-1,8-naphthyridine-3-carboxamide
■ (+)-2-amino-1-éthyl-7-[3-hydroxy-3-(méthoxyméthyl)pent-1-yn-1-yl]-N-méthyl-4-oxo-1,4-dihydro-1,8-naphthyridine-3-carboxamide
■ (-)-2-amino-1-éthyl-7-[3-hydroxy-3-(méthoxyméthyl)pent-1-yn-1-yl]-N-méthyl-4-oxo-1,4-dihydro-1,8-naphthyridine-3-carboxamide
■(+)-2-amino-1-éthyl-7-[3-hydroxy-3-(3-thiényl)but-1-yn-1-yl]-N-méthyl-4-oxo-1,4-dihydro-1,8-naphthyridine-3-carboxamide
■ (-)-2-amino-1-éthyl-7-[3-hydroxy-3-(3-thiényl)but-1-yn-1-yl]-N-méthyl-4-oxo-1,4-dihydro-1,8-naphthyridine-3-carboxamide

Conformément à l'invention, les composés de formule (I) peuvent être préparés selon le procédé présenté dans le schéma 1.

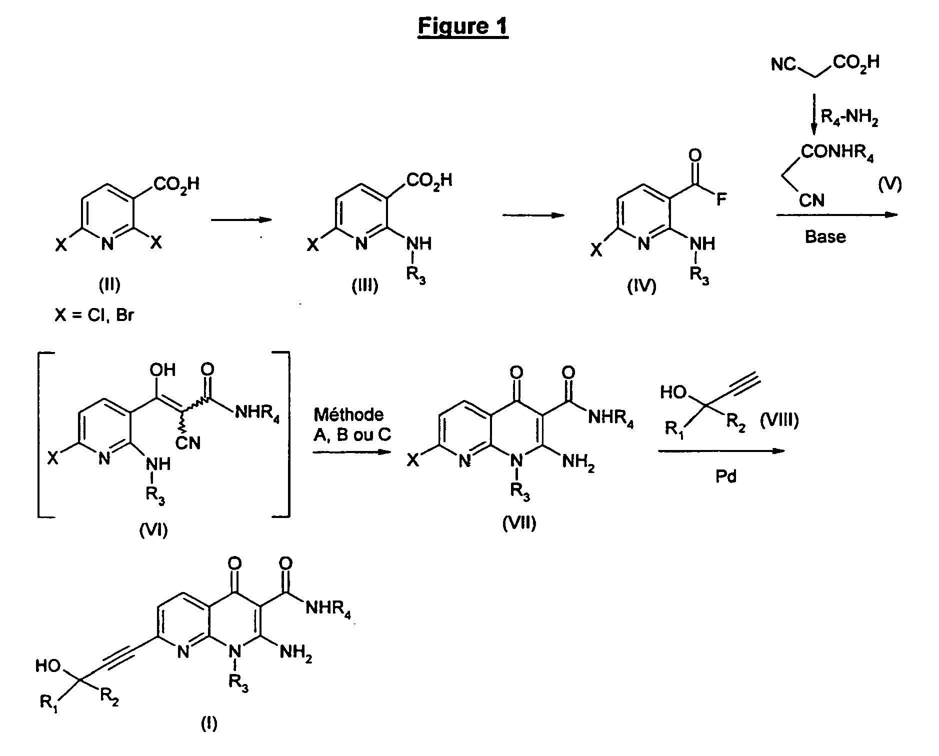

Selon la figure 1, un acide 2,6-dihalogénonicotinique de formule (II) où les groupes X représentent des atomes d'halogène (de préférence le chlore ou le brome), et qui est soit commercial, soit préparé selon les méthodes connues de l'Homme de l'art, est monosubstitué à la position 2 par une amine de formule R₃-NH₂ (où R₃ est tel que défini précédemment en rapport avec les composés de formule (I) objets de l'invention), à une température comprise entre 20°C et 150°C, dans un solvant protique tel qu'un alcool ou l'eau et, éventuellement en tube scellé. On obtient un dérivé 2-aminonicotinique de formule (III), que l'on transforme en fluorure d'acide de formule (IV) par action du fluorure cyanuryle à température ambiante, en présence d'une base telle que la triéthylamine ou la pyridine et dans un solvant inerte tel que le dichlorométhane, comme décrit par G. OLAH et coll. dans Synthesis (1973), 487, ou par d'autres méthodes connues de homme de l'art, telles que celles décrites par MUKAIYAMA et TANAKA dans Chem. Lett. (1976), 303 ou par ISHIKAWA et SASAKI dans Chem. Lett. (1976), 1407. Les fluorures d'acyles de formule (IV), très réactifs mais stables, sont mis ensuite en réaction avec un cyanoacétamide N-substitué de formule (V) en présence d'une base forte telle que l'hydrure de sodium dans un solvant polaire aprotique tel que le diméthylformamide.

Si ron emploie deux équivalents d'hydrure de sodium, après une nuit à température ambiante, on obtient un β-céto-cyanoacétamide de formule (VI), qui est ensuite cyclisé en amino-pyridino[2,3-b]pyridinone de formule (VII) soit par chauffage à une température comprise entre 90 et 125°C dans un solvant polaire tel que le n-butanol, le diméthylsulfoxide ou le diméthylformamyde (méthode A) soit en traitant à température ambiante avec une base forte telle que le tert-butylate de potassium dans un solvant aprotique de préférence le tétrahydrofurane (méthode B).

Lorsque l'on emploie deux équivalents d'hydrure de sodium à l'étape de condensation du dérivé (IV) avec un dérivé (V), puis on introduit un troisième équivalent de NaH après une agitation d'entre 10 et 16 heures à température ambiante, le composé (VI) déprotonné formé se cyclise *in situ* à cette même température avec de bons rendements pour fournir directement l'amino-pyridino[2,3-b]pyridone de formule (VII) (méthode C).

Les N-alkylcyanoacétamides de formule (V) sont préparés en faisant réagir l'acide cyanoacétique avec un chloroformiate d'alkyle (tel qu'éthyle ou isobutyle) en présence d'une base telle que la triéthylamine, à une température inférieure ou égale à 0°C, puis on fait réagir l'intermédiaire anhydride mixte formé avec un excès d'amine de formule R₄-NH₂ (où R₄ est tel que défini précédemment en rapport avec les composés de formule (I) objets de l'invention).

Pour l'obtention d'une pyridino[2,3-b]pyridinone de formule (I), objet de la présente invention, l'intermédiaire halogéné de formule (VII) est couplé selon les méthodes connues de l'Homme de l'art avec un dérivé approprié d'alcool propargylique R₁R₂CH(OH)CCH de formule (VIII) où R₁ et R₂ sont tels que définis pour le composé de formule (I). Par exemple, l'intermédiaire (VII) est engagé dans une réaction de couplage de Sonogashira avec l'alcyne approprié de formule (VIII) en présence de PdCl₂(PPh₃)₂, d'iodure de cuivre de triéthylamine et de diméthylformamide, à une température comprise entre 80°C et 120°C. Cette réaction peut être réalisée dans un tube scellé et sous micro-ondes.

Si nécessaire au cours des étapes réactionnelles présentées dans le schéma 1, le groupe hydroxyle ou certaines fonctions réactives situées sur les groupes R₁, R₂ et R₃ peuvent être protégées temporairement par des groupements protecteurs connus de l'Homme de l'art et tels que décrits dans « Protective Groups in Organic Synthesis », Green et al., 2nd Edition (John Wiley & Sons, Inc., New York).

Dans la figure 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme de l'art.

L'invention, selon un autre de ses aspects, a également pour objet les composés de formule (VII) définis dans la figure 1. Ces composés sont utiles comme intermédiaires de synthèse des composés de formule (I).

Les exemples suivants illustrent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les abréviations et formules brutes suivantes sont utilisées :

| | |
|---|---|
| Cul | iodure de cuivre |
| CH₂Cl₂ | dichlorométhane |
| CLHP | chromatographié liquide haute performance |
| CL/SM | chromatographie liquide/ spectrométrie de masse |
| DMF | diméthylformamide |
| DMSO | diméthyl sulfoxide |
| Et₃N | triéthylamine |
| h | heure(s) |
| HCl | acide chlorhydrique |
| MHz | MegaHertz |
| MeOH | méthanol |
| MgSO₄ | sulfate de magnésium |
| NaCl | chlorure de sodium |
| NH₄Cl | chlorure d'ammonium |
| NH₄OH | hydroxyde d'ammonium |
| NaHCO₃ | hydrogénocarbonate de sodium |
| Na₂SO₄ | sulfate de sodium |
| ppm | partie par million |
| THF | tétrahydrofurane |

### ■ Exemple 1 : 2-amino-1-éthyl-7-(3-hydroxy-3-méthylbut-1-yn-1-yl)-N-méthyl-4-oxo-1,4- dihydro-1,8-naphthyridine-3-carboxamide (Composé n°2)

### 1.1 : Acide 2-(aminoéthyl)-6-chloronicotinique

On agite à température ambiante pendant 72 heures une solution de 18,0 g (84,4 mmoles) d'acide 2,6-dichloronicotinique dans 180 ml d'une solution d'éthylamine à 70 % dans l'eau. L'excès d'amine est alors évaporé sous pression réduite, puis on additionne une solution aqueuse d'acide acétique à 10 % jusqu'à la précipitation du produit. Le solide beige est essoré, rincé avec de l'eau froide et séché à l'étuve. On obtient 10,5 g du produit attendu. Point de fusion : 158-160°C. Rendement = 62 %.

### 1.2 : Fluorure d'acide 2-(aminoéthyl)-6-chloronicotinique

A une suspension de 5,0 g (24,8 mmoles) d'acide 2-(aminoéthyl)-6-chloronicotinique dans 125 ml de dichlorométhane, on ajoute 2 ml (24,8 mmoles) de pyridine et 4,2 ml (49,8 mmoles) de 2,4,6-trifluoro-triazine. On agite le mélange pendant 3 heures à température ambiante puis on filtre. Le solide est rincé avec 50 ml de dichlorométhane et le filtrat est lavé deux fois avec 60 ml d'eau glacée. On sèche la phase organique sur Na₂SO₄ et on évapore le solvant sous pression réduite. On obtient 5,01 g de produit sous forme d'huile orange. Rendement = 99 %.

### 1.3 : N-méthylcyanoacétamide

A une solution refroidie à -30°C de 10,0 g (116,38 mmoles) d'acide cyanoacétique à 99 % et 16,3 ml (116,9 mmoles) de triéthylamine dans 100 ml de THF anhydre, on ajoute goutte à goutte 12,28 ml (128,44 mmoles) de chloroformate d'éthyle puis on agite à -30°C pendant 1 heure et demie. On additionne ensuite goutte à goutte 300 ml de méthanol saturé en méthylamine gazeuse puis on agite à température ambiante pendant une nuit. On évapore les solvants sous pression réduite et on purifie le produit par filtration sur gel de silice en éluant par un mélange dichlorométhane : méthanol (95:5). On obtient 10,0 g de produit sous forme d'un solide beige. Point de fusion = 99°C. Rendement = 87 %

### Méthode A (points 1.4 et 1.5 ci-après).

### 1.4 : 3-[6-chloro-2-(éthylamino)-3-pyridinyl]-2-cyano-3-hydroxy-N-méthyl-2-propènamide

A une solution refroidie à 0-5°C de 9,80 g (100 mmoles) de N-méthylcyanoacétamide dans 100 ml de diméthylformamide anhydre, on ajoute par petites quantités 3,98 g (100 mmoles) d'hydrure de sodium à 60 % dans de l'huile minérale. Après la fin du dégagement d'hydrogène, on agite le mélange pendant 10 minutes à température ambiante, puis on le refroidit à nouveau à 0-5°C. On additionne alors une solution de 10,09 g (49,8 mmoles) de fluorure d'acide 2-(aminoéthyl)-6-chloronicotinique dans 60 ml de diméthylformamide et on agite le milieu à température ambiante pendant une nuit. On additionne 2,85 ml (49,8 mmoles) d'acide acétique et on évapore les volatiles sous pression réduite. Le résidu est repris dans de l'eau et le produit est extrait deux fois avec un mélange dichlorométhane : méthanol (95:5) puis une fois avec un mélange acétate d'éthyle : THF (2:1). Les phases organiques combinées sont séchées sur MgSO₄ puis les solvants sont évaporés sous pression réduite. On obtient 19,0 g de produit utilisé tel quel dans l'étape suivante.

### 1.5 : 2-amino-7-chloro-1-éthyl-N-méthyl-4-oxo-1,4-dihydro-1,8-naphthyridine-3-carboxamide

On chauffe pendant 48 heures à 110°C une solution de 19,0 g du produit brut obtenu à l'issue de l'étape 7.4 (49,8 mmoles) dans 600 ml de n-butanol. On évapore le solvant sous pression réduite et on triture le solide obtenu dans du méthanol. Le solide est ensuite essoré et séché à l'étuve. On obtient 7,9 g du produit attendu sous forme de solide jaune pâle. Point de fusion : 283-286°C. Rendement = 57 %.

### Méthode C (point 1.6 ci-après au lieu de 1.4 et 1.5).

### 1.6 : 2-amino-7-chloro-1-éthyl-N-méthyl-4-oxo-1,4-dihydro-1,8-naphthyridine-3-carboxamide

A une solution refroidie à 0-5 °C de 0,483 g (4,93 mmoles) de N-méthylcyanoacétamide dans 7 ml de diméthylformamide anhydre, on ajoute par petites quantités 0,394 g (9,95 mmoles) d'hydrure de sodium à 60 % dans l'huile minérale. On poursuit l'agitation à cette température pendant dix minutes puis on additionne une solution de 1,0 g (4,93 mmoles) de fluorure d'acide 2-(aminoéthyl)-6-chloronicotinique dans 5 ml de diméthylformamide. On agite le milieu pendant 1 nuit à température ambiante puis on rajoute par petites quantités 0,197 g (4,93 mmoles) d'hydrure de sodium à 60 %. On poursuit l'agitation à cette température pendant 10 minutes puis on additionne 0,56 ml (9,78 mmoles) d'acide acétique. On ajoute ensuite 60 ml d'eau et on essore le solide que l'on rince à l'eau puis on le sèche à l'étuve. On obtient 1,30 g du produit attendu. Point de fusion : 283-284°C. MH⁺ = 281. Rendement = 94 %.
RMN 1H (DMSO-d6, 400MHz) : 8 11,75 (s, < 1H, très élargi); 11,00 (q, 1H, élargi); 8;45 (d, 1H); 8,10 (s, 1H élargi); 7,40 (d, 1H); 4,40 (q, 2H); 2,80 (d, 3H); 1,25 (t, 3H).

### 1.7: 2-amino-1-éthyl-7-(3-méthyl-3-triméthylsilanyloxy-but-1-yn-1-yl)-N-méthyl-4-oxo-1,4- dihydro-1,8-naphthyridine-3-carboxamide

Dans un tricol de 2 1, on introduit successivement 40,7 g de 2-amino-7-chloro-1-éthyl-N-méthyl-4-oxo-1,4-dihydro-[1,8]naphthyridine-3-carboxamide (0,145 mole) et 56,2 ml de [(1,1-diméthyl-2-propynyl)-oxy]triméthylsilane (0,290 mole) dans un mélange de 365 ml de diméthylformamide et 365 ml de triéthylamine. On fait barboter de l'argon pendant 15 minutes dans le mélange réactionnel puis on ajoute successivement 0,983 g de Cul (5,16 mmoles) et 5,1 g de bis(triphénylphosphine) palladium(II)dichloride (7,2 mmoles). Le mélange réactionnel est chauffé pendant 15 heures à 90°C puis concentré sous vide réduit. Le résidu est repris dans 500 ml d'un mélange acétate d'éthyle/NaHCO_{3aq} (V/V = 1/1) et filtré sur un tampon de célite avec rinçage à l'acétate d'éthyle. La phase aqueuse est extraite au dichlorométhane (3 x 50 ml) puis les phases organiques sont réunies, séchées sur sulfate de sodium, filtrées et concentrées sous vide. Le brut de réaction est purifié par chromatographie sur colonne de silice (élution avec un gradient dichlorométhane : acétate d'éthyle, 80 : 20 à 50 : 50 puis un gradient dichlorométhane : méthanol, 95 : 5 à 90 : 10) pour donner 36,2 g de produit attendu (rendement = 62,3%) ainsi que 7,4 g de produit désilylé décrit dans l'étape suivante (rendement = 15,5%).

### 1.8 : 2-amino-1-éthyl-7-(3-hydroxy-3-méthylbut-1-yn-1-yl)-N-méthyl-4-oxo-1,4-dihydro-1,8-naphthyridine-3-carboxamide

On dissout 15 g de 2-amino-1-éthyl-7-(3-méthyl-3-triméthylsilanyloxy-but-1-yn-1-yl)-N-méthyl-4-oxo-1,4-dihydro-1,8-naphthyridine-3-carboxamide (37,4 mmoles) dans 815 ml de tétrahydrofurane. On refroidit la solution à 0°C puis on ajoute 13,0 g de fluorure de tétrabutylammonium, trihydrate (41,2 mmoles). On agite le mélange réactionnel pendant 15 minutes à température ambiante. On évapore à sec et on reprend le résidu dans un mélange acétate d'éthyleltétrahydrofurane/eau. La phase aqueuse est extraite au dichlorométhane puis les phases organiques sont regroupées, séchées sur sulfate de sodium, filtrées et concentrées sous vide. On obtient 15 g de produit brut qui sont solubilisés à chaud dans un 1 l de méthanol puis on ajoute 6,75 g de charbon activé et on agite le mélange à 70°C pendant 5 heures. On filtre sur célite et après évaporation sous vide on obtient 11,9 g de produit attendu sous forme d'un solide blanc.
Point de fusion = 255°C. MH⁺= 329. Rendement = 82,9 %.
RMN 1H (DMSO-d6, 400MHz, δ en ppm) : δ 11,75 (s, < 1H, très élargi); 11,00 (q, 1H, élargi); 8;40 (d, 1H); 8,00 (s, 1H élargi); 7,40 (d, 1H); 5,62 (s, 1H, élargi); 4,4 (q, 2H); 2,75 (d, 3H); 1,45 (s, 6H); 1,20 (t, 3H).

### ■ Exemple 2: (±)-2-amino-1-éthyl-7-1(3-hydroxytétrahydrofuran-3-yl)éthynyl]-N-méthyl-4-oxo-1,4-dihydro-1,8-naphthyridine-3-carboxamide (Composé n°7)

### 2.1 : (±)-3-éthynyltétrahydro-3-furanol

Une solution de 3-oxotétrahydrofurane (4,97 mmoles; préparation décrite dans Tetrahedron 1991, 47, 6975-6982) dans 20 ml de tétrahydrofurane est ajoutée à 20 ml d'une solution commerciale de bromure d'éthynylmagnésium 0,5 M dans le tétrahydrofurane (10 mmoles, Aldrich) préalablement refroidie à 0°C. On agite ensuite 4 heures à température ambiante et on ajoute une solution de NH₄Cl_{aq} saturée. On extrait à l'acétate d'éthyle puis les phases organiques sont réunies, lavées avec une solution de NaCl_{aq} saturée, séchées sur sulfate de sodium, filtrées et concentrées sous vide. Après purification par chromatographie sur colonne de silice, on obtient 319 mg (2,79 mmoles) de (±)-3-éthynyltétrahydro-3-furanol sous forme d'une huile jaune avec un rendement de 57%.

### 2.2 : (±)-2-amino-1-éthyl-7-[(3-hydroxytétrahydrofuran-3-yl)éthynyl]-N-méthyl-4-oxo-1,4-dihydro-1,8-naphthyridine-3-carboxamide

Dans un tube pour micro-ondes de 10 ml, on place une suspension de 0,3 g (2,24 mmoles) de 2-amino-7-chloro-1-éthyl-N-méthyl-4-oxo-1,4-dihydro-[1,8]naphthyridine-3-carboxamide dans 17 ml d'un mélange diméthylformamide/triéthylamine (V/V; 2/1). On fait barboter de l'argon dans cette suspension pendant 10 minutes puis on ajoute successivement 0,319 g (2,79 mmoles) de (±)-3-éthynyltétrahydro-3-furanol, 0,043 g de Cul (0,23 mmole) et 0,079 g de bis(triphénylphosphine) palladium(II)dichloride (0,11 mmole).

On place le tube scellé dans le four à micro-ondes (appareil CEM, modèle Discover) et le mélange est chauffé sous pression à 90°C pendant 60 minutes (P = 100W) puis refroidi et évaporé à sec. On reprend avec de l'acétate d'éthyle et la phase organique est lavée successivement avec NaHCO_{3aq} puis NaCl_{aq} saturées, séchée sur sulfate de sodium ; filtrée et concentré sous vide. Le résidu obtenu est purifié par chromatographie sur silice (élution avec un gradient dichlorométhane : méthanol, 98: 2 à 95: 5). On obtient 0,412 g du produit attendu sous forme d'un solide jaune pâle.
Point de fusion = 253°C. MH⁺ = 357. Rendement = 52 %.
RMN 1H (DMSO-d6, 400MHz, δ en ppm) : δ 11,75 (s, < 1H, très élargi); 11,00 (q, 1H, élargi); 8,45 (d, 1H); 8,00 (s, 1H élargi); 7,4 (d, 1H); 5,6 (d, 1H); 4,55-4,30 (m, 3H); 3,95-3,80 (m, 4H); 2,8 (d, 3H); 2,20 (m, 1 H); 1,9-1,35 (m, 8 H); 1,25 (t, 3H).

### ■ Exemple 3: (±)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide (Composé n°8)

### 3.1 : Préparation du (±)-1-méthoxy-2-méthyl-3-butyn-2-ol

Dans un tricol sous argon, on coule 1400 ml (0,7 mole) d'une solution commerciale de chlorure (ou bromure) d'éthynyl magnésium 0,5 M dans le tétrahydrofurane. On refroidit à 2°C avec un bain de glace et on ajoute lentement la solution de 30 g (0,327 mole) de méthoxyacétone dans 600 ml de tétrahydrofurane (exothermique). On agite pendant 1 heure à 2°C puis on verse sur un mélange glace/NH₄Cl_{aq} saturée. On extrait à l'éther puis les phases organiques sont réunies, séchées sur sulfate de sodium, filtrées et concentrées sous vide limité. On obtient le produit attendu sous forme d'une huile marron de 38 g (rendement brut quantitatif) qui est utilisée sans purification ultérieure dans l'étape suivante.

### 3.2: (±)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide

Dans un tube pour micro-ondes de 80 ml, on place une suspension de 3 g (10,69 mmoles) de 2-amino-7-chloro-1-éthyl-N-méthyl-4-oxo-1,4-dihydro-[1,8]naphthyridine-3-carboxamide dans 20 ml d'un mélange DMF/Et₃N(V/V ; 1/1). On fait barboter de l'argon dans cette suspension pendant 10 minutes puis on ajoute successivement 1,83 g de (±)-1-méthoxy-2-méthyl-3-butyn-2-ol (16,03 mmoles), 0,081 g de Cul (0,43 mmole) et 0,375 g de bis(triphénylphosphine) palladium(II)dichloride (0,53 mmole).

On place le tube scellé dans le four à micro-ondes (appareil CEM, modèle Discover) et le mélange est chauffé sous pression à 90°C pendant 60 minutes (P = 100W) puis refroidi et évaporé à sec. Le résidu est repris dans un mélange acétate d'éthyle/THF puis lavé avec une solution aqueuse HCl 0,1N. La phase organique est séchée sur sulfate de sodium, filtrée et concentrée sous vide.

Le résidu obtenu est purifié par chromatographie sur silice (dépôt solide; élution avec un gradient cyclohexane: acétate d'éthyle, 30 : 70 à 20 : 80). On obtient 2,49 g du produit attendu sous forme d'un solide jaune pâle.

Le produit peut-être recristallisé dans l'éthanol pour donner des cristaux blancs.
Point de fusion = 211 °C. MH⁺ = 358. Rendement = 65 %.
RMN 1H (DMSO-d6, 400MHz, δ en ppm) : δ 11,75 (s, < 1H, très élargi); 11,00 (q, 1H, élargi): 8,45 (d, 1H); 8,00 (s, 1H élargi); 7,4 (d, 1H); 5,8 (s, 1H); 4,4 (q, 2H); 3,5-3,3 (m+s, 5H); 2,8 (d, 3H); 1,45 (s, 3H); 1,2 (t, 3H).

### ■ Exemple 4 : (±)-2-amino-7-(3-cyclopentyl-3-hydroxyprop-1 -yn-1-yl)-1-éthyl-N-méthyl-4-oxo-1,4-dihydro-1,8-naphthyridine-3-carboxamide (Composé n°10)

### 4.1 : (±)-1-cyclopentylprop-2-yn-1-ol

Une solution de 0,87 ml de cyclopentanecarboxaldehyde (8,15 mmoles ; Aldrich) dans 10 ml de tétrahydrofurane est ajouté à 18 ml d'une solution commerciale de bromure d'éthynylmagnésium 0,5 M dans le tétrahydrofurane (10 mmoles, Aldrich) préalablement refroidie à 0°C. On agite ensuite 2 heures à température ambiante et on ajoute une solution de NH₄Cl_{aq} saturée. On extrait à l'acétate d'éthyle puis les phases organiques sont réunies, lavées avec une solution de NaCl_{aq} saturée, séchées sur sulfate de sodium, filtrées et concentrées sous vide. On obtient 0,931 g de (±)1-cyclopentylprop-2-yn-1-ol sous forme d'une huile marron clair avec un rendement de 92%.

### 4.2 : (±)-2-amino-7-(3-cyclopentyl-3-hydroxyprop-1-yn-1-yl)-1-éthyl-N-méthyl-4-oxo-1,4-dihydro-1,8-naphthyridine-3-carboxamide

Dans un tube pour micro-ondes de 10 ml, on place une suspension de 1 g (3,56 mmoles) de 2-amino-7-chloro-1-éthyl-N-méthyl-4-oxo-1,4-dihydro-[1,8]naphthyridine-3-carboxamide dans 25 ml d'un mélange diméthylformamide/triéthylamine (V/V ; 2.5/1). On fait barboter de l'argon dans cette suspension pendant 10 minutes puis on ajoute successivement 0,931 g (7,5 mmoles) de (±)-1-cyclopentylprop-2-yn-1-ol, 0,068 g de Cul (0,36 mmole) et 0,125 g de bis(triphénylphosphine) palladium(II)dichloride (0,18 mmole).

On place le tube scellé dans le four à micro-ondes (appareil CEM, modèle Discover) et le mélange est chauffé sous pression à 80°C pendant 17 minutes (P = 50 W). On évapore à sec puis on reprend avec de l'acétate d'éthyle. La phase organique est lavée successivement avec NaHCO_{3aq} puis NaCl_{aq} saturées, séchée sur sulfate de sodium, filtrée et concentré sous vide. Le résidu obtenu est purifié par chromatographie sur silice (élution avec un gradient dichlorométhane : méthanol, 98 : 2 à 95 : 5). On obtient 0,73 g du produit attendu sous forme d'un solide jaune pâle.
Point de fusion = 253°C. MH⁺ = 369. Rendement = 56 %.
RMN 1H (DMSO-d6, 400MHz, δ en ppm) : δ 11,75 (s, < 1H, très élargi); 11,00 (q, 1H, élargi); 8,45 (d, 1H); 8,00 (s, 1H élargi), 7,45 (d, 1H); 6,05 (s, 1H); 4,4 (q, 2H); 3,95-3,80 (m, 4H); 2,8 (d, 3H); 2,35-2,10 (m, 2 H); 1,45 (s, 3H); 1,25 (t, 3H).

### ■ Exemple 5 : (±)-2-amino-7-(3-hydroxy-4-méthoxy-3-méthylbut-1-yn-1-yl)-N-méthyl-4-oxo-1-(pyridin-2-ylméthyl)-1,4-dihydro-1,8-naphthyridine-3-carboxamide (Composé n°47)

### 5.1 : acide 6-chloro-2-[(pyridin-2-ylméthyl)amino]nicotinique

Dans un tube scellé de 80 ml contenant 40 ml de tert-butanol, on place 4 g d'acide 2,6-dicloronicotinique (20,83 mmoles, Aldrich) et 10,74 ml de 2-(aminométhyl)pyridine (104,17 mmoles). On chauffe à 100 °C pendant une nuit et on évapore à sec. On reprend le résidu dans l'eau et on acidifie en versant une solution aqueuse d'acide acétique à 10%. On isole le précipité par filtration et après séchage à l'étuve sous vide réduit, on obtient 4,35 g de produit attendu sous forme de poudre jaune (rendement = 79%).

### 5.2 : fluorure de 6-chloro-2-[(pyridin-2-ylméthyl)amino]nicotinoyle

A une suspension de 4,3 g d'acide 6-chloro-2-[(pyridin-2-ylméthyl)amino]nicotinique (16,31 mmoles) dans 90 ml de dichlorométhane, on ajoute successivement 2,27 ml de triéthylamine (16,31 mmoles) et 1,65 ml de 2,4,6-trifluoro-triazine (19,57 mmoles). On agite le mélange pendant 1 heure à température ambiante puis on ajoute un mélange dichlorométhane/eau glacée. La phase organique est lavée 3 fois avec NaHCO_{3aq} glacée puis séchée sur Na₂SO₄, filtrée et concentrée sous vide. On obtient 3,97 g de produit attendu sous forme d'un solide orange. Rendement = 92 %.

### 5.3 : 2-amino-7-chloro-N-méthyl-4-oxo-1-(pyridin-2-ylméthyl)-1,4-dihydro-1,8-naphthyridine-3-carboxamide

A une solution refroidie à 0-5 °C de 1,31 g (13,36 mmoles) de N-méthylcyanoacétamide (préparé selon 1.3) dans 20 ml de diméthylformamide anhydre, on ajoute par fractions 1,12 g (28.06 mmoles) d'hydrure de sodium à 60 % dans l'huile minérale. On poursuit l'agitation à cette température pendant dix minutes puis on additionne une solution de 3,55 g (4,93 mmoles) de fluorure de 6-chloro-2-[(pyridin-2-ylméthyl)amino]nicotinoyle dans 20 ml de diméthylformamide. On agite le milieu pendant 1 nuit à température ambiante puis on rajoute par fractions 0,561 g (14,03 mmoles) d'hydrure de sodium à 60 %. On poursuit l'agitation à cette température pendant 1 heure minutes puis verse le mélange réactionnel sur de la glace et on acidifie jusqu'à pH 5-6 avec une solution aqueuse d'acide acétique à 10%. On isole le précipité par filtration et après séchage à l'étuve, on obtient 3,98 g du produit attendu sous forme d'un solide beige. MH⁺ = 344. Rendement = 87 %.
RMN 1H (DMSO-d6, 400MHz, δ en ppm) : δ 9,20 (s, < 1H, très élargi); 8.85 (s, 1H); 8,30 (m, 1H); 8,10-7,85 (m, 2H, élargi); 7,80 (d, 1H): 7,75 (s, 1H, élargi); 6,90 (d, 1H); 4,90 (s, 2H); 2,95 (s, 3H).

### 5.4 : Chlorhydrate de (±)-2-amino-7-(3-hydroxy-4-méthoxy-3-méthylbut-1-yn-1-yl)-N-méthyl-4-oxo-1-(pyridin-2-ylméthyl)-1,4-dihydro-1,8-naphthyridine-3-carboxamide

Dans un tube pour micro-ondes de 80 ml, on place une suspension de 1 g de 2-amino-7-chloro-N-méthyl-4-oxo-1-(pyridin-2-ylméthyl)-1,4-dihydro-1,8-naphthyridine-3-carboxamide (2,91 mmoles) dans un mélange de 20 ml de diméthylformamide et 5,7 ml de triéthylamine. On fait barboter de l'argon dans cette suspension puis on ajoute successivement 0,498 g de (±)-1-methoxy-2-méthyl-3-butyn-2-ol (±)(4.36 mmoles), 0,055g de Cul (0,29 mmole) et 0,102 g de bis(triphénylphosphine)palladium(II)dichloride (0,15 mmole).

On place le tube scellé dans le four à micro-ondes (appareil CEM, modèle Discover) et le mélange est chauffé sous pression à 80°C pendant 45 minutes (P = 100W) puis refroidi et évaporé à sec. Le résidu est repris dans l'acétate d'éthyle et l'eau. La phase aqueuse est extraite à l'acétate d'éthyle (3 fois), puis les phases organiques sont réunies, séchées sur sulfate de sodium, filtrées et concentrées sous vide.

Le résidu obtenu est purifié par chromatographie sur silice (dépôt solide ; élution gradient CH₂Cl₂/MeOH/NH₄OHaq, 98/2/0,2 à 95/5/0,5). Après trituration dans l'éther, on obtient 0,425 g de produit attendu sous forme d'un solide jaune.
Point de fusion = 169°C. MH⁺ = 422. Rendement = 35 %.
RMN 1H (DMSO-d6, 400MHz, δ en ppm) : δ 11,65 (s, < 1H, très élargi); 11,00 (q, 1H, élargi); 8,50-8,35 (m, 2H); 8,00 (s, 1H, élargi); 7,75 (ddd, 1H); 7,45-7,20 (m, 3H); 5,80 (s, 2H, élargi); 5,70 (s, 1H); 3,45-3,25 (m+s, 5H); 2,80 (d, 3H); 1,40 (s, 3H).

### ■ Exemple 6 : (±)-2-amino-1-éthyl-7-(3-hydroxy-3-pyrazin-2-ylbut-1-yn-1-yl)-N-méthyl-4-oxo-1,4-dihydro-1,8-naphthyridine-3-carboxamide (Composé n°48)

### 6.1 : (±)-2-pyrazin-2-ylbut-3-yn-2-ol

Une solution de chlorure d'éthynylmagnésium 0,5 M dans le tétrahydrofurane (31,90 mmoles, Aldrich) est diluée avec 50 ml de tétrahydrofurane puis refroidie à 0°C. On additionne ensuite en plusieurs fractions, 3 g de 1-pyrazin-2-yléthanone (24,56 mmoles, Lancaster) et on agite à 0°C pendant 4 heures. On refroidit à nouveau avec un bain de glace et on ajoute lentement une solution de NH₄Cl_{aq}. On extrait 2 fois à l'acétate d'éthyle puis les phases organiques sont réunies, séchées sur sulfate de sodium, filtrées et concentrées sous vide. Le résidu huileux obtenu est purifié par chromatographie sur silice (élution avec un gradient cyclohexane : acétate d'éthyle, 70 : 30 à 50 : 50) et on obtient 2,9 g du produit attendu sous forme d'une huile jaune (rendement = 80%).

### 6.2 : (±)-2-amino-1-éthyl-7-(3-hydroxy-3-pyrazin-2-ylbut-1-yn-1-yl)-N-méthyl-4-oxo-1,4-dihydro-1,8-naphthyridine-3-carboxamide

Dans un tube pour micro-ondes de 10 ml, on place une suspension de 1 g (3,56 mmoles) de 2-amino-7-chloro-1-éthyl-N-méthyl-4-oxo-1,4-dihydro-[1,8]naphthyridine-3-carboxamide dans 25 ml d'un mélange DMF/Et₃N (V/V ; 2.5/1). On fait barboter de l'argon dans cette suspension pendant 10 minutes puis on ajoute successivement 0,792 g de (±)-2-pyrazin-2-ylbut-3-yn-2-ol (5,34 mmoles), 0,068 g de Cul (0,36 mmole) et 0,125 g de bis(triphénylphosphine) palladium(II)dichloride (0,18 mmole).

On place le tube scellé dans le four à micro-ondes (appareil CEM, modèle Discover) et le mélange est chauffé sous pression à 80°C pendant 2 x 45 minutes (P = 50 W). Après retour à l'ambiante, on évapore à sec puis on reprend avec de l'acétate d'éthyle. La phase organique est lavée successivement avec NaHCO_{3aq} puis NaCl_{aq} saturées, séchée sur sulfate de sodium; filtrée et concentré sous vide. Le résidu obtenu est purifié par chromatographie sur silice (élution avec un gradient dichlorométhane : méthanol, 100: 0 à 98: 2). On obtient 0,2 g du produit attendu impur sous forme d'un solide marron. On effectue une nouvelle purification par chromatographie sur colonne de silice (élution avec un gradient cyclohexane : acétate d'éthyle, 20 : 80 à 0 : 100) et on obtient finalement 0,068 g de produit attendu sous forme d'un solide jaune.
Point de fusion = 271 °C. MH⁺ = 393. Rendement = 4.9 %.
RMN 1H (DMSO-d6, 400MHz, δ en ppm) : δ 11,75 (s, < 1H, très élargi); 11,00 (q, 1H, élargi); 9,0 (s, 1H); 8,65 (m, 1H); 8,40 (d, 1H); 8,00 (s, 1H élargi); 7,40 (d, 1H); 6,85 (s, 1H); 4,4 (q, 2H); 2,75 (d, 3H); 1,80 (s, 3H); 1,20 (t, 3H).

### ■ Exemple 7 : (±)-2-amino-7-(3-cyclopropyl-3-hydroxy-4-méthoxybut-1-yn-1-yl)-1-éthyl-N-méthyl-4-oxo-1,4-dihydro-1,8-naphthyridine-3-carboxamide

### (Composé n°50)

### 7.1 : (±)-2-cyclopropyl-1-méthoxybut-3-yn-2-ol

### 7.1.1 : N-méthoxy-N-méthyl-2-méthoxyacétamide

A une solution de chlorhydrate de N-méthyl-O-méthylhydroxylamine dans 250 ml de dichlorométhane refroidie à 0°C, on ajoute 35,96 ml de triéthylamine (258 mmoles) puis une solution de 14 g de chlorure de méthoxyacétyle (Aldrich, 129 mmoles) dans 250 ml de dichlorométhane. On agite pendant 3 heures en laissant remonter à température ambiante. On ajoute une solution de HCl_{aq} 1 N puis la phase organique est lavée à l'eau, séchée sur sulfate de sodium, filtrée et concentrée sous vide. On obtient 13,14 g de produit attendu sous forme d'une huile qui est utilisée sans purification ultérieure (rendement brut = 76%).

### 7.1.2 : 1-méthoxy-4-(triméthylsilyl)but-3-yn-2-one

Une solution de 14,75 ml de triméthylsilylacétylène (103,62 mmoles) dans 250 ml de tétrahydrofurane est refroidie à -70°C puis on ajoute 64,76 ml d'une solution de n-BuLi 1,6 M (103,62 mmoles) dans l'hexane. On agite 25 minutes à - 70°C puis on ajoute rapidement une solution de 13,14 g de N-méthoxy-N-méthyl-2-méthoxyacétamide (98,69 mmoles) dans 250 ml de tétrahydrofurane. Lors de l'addition, la température remonte jusqu'à -50°C puis on retire le bain froid et on agite durant 2 heures à température ambiante. On ajoute une solution de HCl_{aq} 1 N puis on extrait 4 fois à l'acétate d'éthyle. Les phases organiques sont réunies, séchées sur sulfate de sodium, filtrées et concentrées sous vide. On obtient 13,4 g de 1-méthoxy-4-(triméthylsilyl)but-3-yn-2-one sous forme d'une huile marron clair que l'on utilise sans purification ultérieure (rendement brut = 80%).

### 7.1.3 : (±)-2-cyclopropyl-1 -méthoxy-4-(triméthylsilyl)but-3-yn-2-ol

A une solution de 3 g de 1-méthoxy-4-(triméthylsilyl)but-3-yn-2-one (17,62 mmoles) dans 160 ml d'éther, on ajoute 52,85 ml de bromure de cyclopropylmagnésium 0,5 M (26,43 mmoles, Aldrich). On agite à température ambiante pendant une nuit puis on refroidit le mélange avec un bain eau/glace et on ajoute une solution de NH₄Cl_{aq} et de l'acétate d'éthyle. On extrait 3 fois à l'acétate d'éthyle puis les phases organiques sont réunies, séchées sur sulfate de sodium, filtrées et concentrées sous vide. Le résidu huileux est purifié par chromatographie sur colonne de silice en éluant avec un gradient cyclohexane : acétate d'éthyle, 100 : 0 à 80: 20 pour obtenir 1,9 g de (±)-2-cyclopropyl-1-méthoxy-4-(triméthylsilyl)but-3-yn-2-ol sous forme d'huile avec un rendement de 51 %.

### 7.1.4 : (±)-2-cyclopropyl-1-méthoxybut-3-yn-2-ol

A une solution de 1,67 g de (±)-2-cyclopropyl-1-méthoxy-4-(triméthylsilyl)but-3-yn-2-ol (7,86 mmoles) dans le méthanol, on ajoute 10,9 mg de carbonate de potassium (0,08 mmole) et on agite une nuit à température ambiante. On évapore le méthanol et on reprend avec un mélange acétate d'éthyle/eau. On extrait 3 fois à l'acétate d'éthyle puis les phases organiques sont réunies, séchées sur sulfate de sodium, filtrées et concentrées sous vide. On obtient 0,434 g de (±)-2-cyclopropyl-1-méthoxybut-3-yn-2-ol sous forme d'huile utilisée sans purification ultérieure (rendement brut = 40 %).

### 7.2 : (±)-2-amino-7-(3-cyclopropyl-3-hydroxy-4-méthoxybut-1-yn-1-yl)-1-éthyl-N-méthyl-4-oxo-1,4-dihydro-1,8-naphthyridine-3-carboxamide

Dans un tube pour micro-ondes de 10 ml, on place une suspension de 0,47 g (1,68 mmoles) de 2-amino-7-chloro-1-éthyl-N-méthyl4-oxo-1,4-dihydro-[1,8]naphthyridine-3-carboxamide dans un mélange de 8,4 ml de diméthylformamide et 3 ml de triéthylamine. On fait barboter de l'argon dans cette suspension pendant 10 minutes puis on ajoute successivement 0,433 g de de (±)-2-cyclopropyl-1-méthoxybut-3-yn-2-ol (3,09 mmoles), 0,032 g de Cul (0,17 mmole) et 0,059 g de bis(triphénylphosphine) palladium(II)dichloride (0,08 mmole).

On place le tube scellé dans le four à micro-ondes (appareil CEM, modèle Discover) et le mélange est chauffé sous pression à 80°C pendant 30 minutes (P = 50 W) puis refroidi et évaporé à sec. On reprend avec de l'acétate d'éthyle et la phase organique est lavée successivement avec NaHCO_{3aq} puis NaCl_{aq} saturées, séchée sur sulfate de sodium ; filtrée et concentrée sous vide. Le résidu obtenu est purifié par chromatographie sur silice en éluant avec un gradient dichlorométhane : méthanol, 100 : 0 à 97 : 3. Après trituration dans l'éther et filtration, on obtient 0,316 g du produit attendu sous forme d'un solide jaune pâle.
Point de fusion = 208 °C. MH⁺ = 385. Rendement = 49 %.
RMN 1H (DMSO-d6, 400MHz, δ en ppm) : δ 11,75 (s, < 1H, très élargi); 11,00 (q, 1H, élargi); 8,45 (d, 1H); 8,00 (s, 1H élargi); 7,40 (d, 1H); 5,60 (s, 1H); 4,4 (q, 2H); 3,50 (s, 2H); 3,40 (s, 3H); 2,75 (d, 3H); 1,20 (t, 3H); 0,6-0,3 (m, 4H).

### ■ Exemple 8 : (±)-2-amino-1-cyclopentyl-7-(3,4-dihydroxy-3-méthylbut-1-yn-1-yl)-N-méthyl-4-oxo-1,4-dihydro-1,8-naphthyridine-3-carboxamide (Composé n°53)

### 8.1 : (±)-2-méthylbut-3-yne-1,2-diol

Une solution commerciale de chlorure d'éthynylmagnésium 0,5 M dans le tétrahydrofurane est diluée avec 200 ml de tétrahydrofurane puis refroidie à 0 °C. On ajoute ensuite une solution d'hydroxyacétone dans 200 ml de tétrahydrofurane et on agite à température ambiante pendant 3 heures. On refroidit le mélange réactionnel puis on ajoute une solution de NH₄Claq. On extrait 3 fois à l'acétate d'éthyle puis les phases organiques sont réunies, séchées sur sulfate de sodium, filtrées et concentrées sous vide (environ 200 mbar). On obtient finalement 20 g de produit attendu sous forme d'une huile marron qui est utilisée sans purification ultérieure (rendement brut quantitatif).

### 8.2 : Acide 2-(aminocyclopentyl)-6-chloronicotinique

Dans un tube scellé contenant 40 ml de tert-butanol, on place 15 g d'acide 2,6-dicloronicotinique (70,31 mmoles, Aldrich) et 34,7 ml de cyclopentylamine (351,56 mmoles). On chauffe à 100 °C pendant une nuit et on évapore à sec. On reprend le résidu dans l'eau et on acidifie en versant une solution aqueuse d'acide acétique à 10% puis on extrait au choloroforme. Les phases organiques sont réunies, séchées sur sulfate de sodium, filtrées et concentrées sous vide. Le résidu solide est trituré dans un mélange éther/pentane pour donner 5 g de produit attendu sous forme d'un solide blanc cassé (rendement = 29,5%).

### 8.3 : Fluorure d'acide 2-(aminocyclopentyl)-6-chloronicotinique

A une suspension de 1,0 g (4,15 mmoles) d'acide 2-(aminocyclopentyl)-6-chloronicotinique dans 12 ml de dichlorométhane, on ajoute 0,34 ml (4,15 mmoles) de pyridine et 0,53 ml (6,23 mmoles) de 2,4,6-trifluoro-triazine. On agite le mélange pendant 2 heures à température ambiante puis on dilue avec du dichlorométhane. La phase organique est lavée 2 fois avec une solution de NaHCO₃aq glacée puis séchée sur Na₂SO₄. filtrée et concentrée sous pression réduite. On obtient 1 g de produit sous forme d'huile marron qui est engagé immédiatement dans l'étape suivante. Rendement brut quantitatif.

### Méthode A (points 8.4 ci-après).

### 8.4 : 3-[6-chloro-2-(cyclopentylamino)-3-pyridinyl]-2-cyano-3-hydroxy-N-méthyl-2-propènamide + 2-amino-7-chloro-1-cyclopentyl-N-méthyl-4-oxo-1,4-dihydro-1,8-naphthyridine-3-carboxamide.

A une solution refroidie à 0-5°C de 0,427 g (4,36 mmoles) de N-méthylcyanoacétamide (préparation décrite en 1.3) dans 10 ml de DMF anhydre, on ajoute par petites quantités 0,349 g (8,72 mmoles) d'hydrure de sodium à 60 % dans de l'huile minérale. Après la fin du dégagement d'hydrogène, on agite le mélange pendant 10 minutes à température ambiante, puis on le refroidit à nouveau à 0-5°C. On additionne alors une solution de 1,0 g (4,15 mmoles) de fluorure d'acide 2-(aminocyclopentyl)-6-chloronicotinique dans 10 ml de DMF et on agite le milieu à température ordinaire pendant 1 heure. On additionne 0,174 mg (4,35 mmoles) d'hydrure de sodium à 60 % dans de l'huile minérale et on agite à température ambiante pendant une nuit. On verse le mélange réactionnel sur un mélange glace/HCl_{aq} 0,1 N et on isole le précipité par filtration. Après rinçage à l'eau et séchage à l'étuve on obtient un solide verdâtre de 1,2 g constitué d'un mélange 2/1 de 3-[6-chloro-2-(cyclopentylamino)-3-pyridinyl]-2-cyano-3-hydroxy-N-méthyl-2-propènamide et de 2-amino-7-chloro-1-cyclopentyl-N-méthyl-4-oxo-1,4-dihydro-1,8-naphthyridine-3-carboxamide.

### Méthode B (point 8.5 ci-après).

### 8.5 : 2-amino-7-chloro-1-cyclopentyl-N-méthyl-4-oxo-1,4-dihydro-1,8-naphthyridine-3-carboxamide

A une solution de 1,2 g du mélange décrit en 8.4 (3,74 mmoles) dans 40 ml de tétrahydrofurane anhydre, on ajoute une solution commerciale de tert-butylate de potassium 1,0 M dans le tétrahydrofurane (7,48 mmoles ; Aldrich). On agite 15 minutes à température ambiante puis on ajoute une solution aqueuse de HCl_{aq} 0,1N et on extrait 2 fois à l'acétate d'éthyle. Les phases organiques sont séchées sur sulfate de sodium, filtrées et concentrées sous vide. Après trituration dans l'éther et séchage à l'étuve on obtient 0,53 g de produit attendu sous forme d'un solide jaune (rendement = 44%). Point de fusion = 256-258°C. MH⁺ = 321.

### 8.6: (±)-2-amino-1-cyclopentyl-7-(3,4-dihydroxy-3-méthylbut-1-yn-1-yl)-N-methyl-4-oxo-1,4-dihydro-1,8-naphthyridine-3-carboxamide

Dans un tricol de 500 ml, on introduit successivement 15 g de 2-amino-7-chloro-1-cyclopentyl-N-méthyl-4-oxo-1,4-dihydro-[1,8]naphthyridine-3-carboxamide (53,44 mmoles) et 11,11 g de (±)-2-methylbut-3-yne-1,2-diol (83,39 mmoles) dans un mélange de 267 ml de diméthylformamide et 104 ml de triéthylamine. On fait barboter de l'argon pendant 15 minutes dans le mélange réactionnel puis on ajoute successivement 1,02 g de Cul (5,34 mmoles) et 1,87 g de bis(triphénylphosphine) palladium(II)dichloride (2,67 mmoles). Le mélange réactionnel est chauffé pendant 30 minutes à 100 °C puis concentré sous vide réduit. Le résidu est repris dans un mélange chloroforme/eau et on filtre l'insoluble. On obtient 20 g d'un solide que l'on purifie par chromatographie sur silice (dépôt solide après solubilisation dans un mélange tétrahydrofurane/méthanol puis élution avec un gradient dichlorométhane/méthanol, 99 : 1 à 80 :20) pour donner 7,5 g de produit attendu sous forme d'un solide beige.
Point de fusion = 210.5°C. MH⁺ = 385. Rendement = 42 %.
RMN 1H (DMSO-d6, 400MHz, δ en ppm) : δ 11,6 (s, < 1H, très élargi); 11,05 (q, 1H, élargi); 8,40 (d, 1H); 7,35 (d, 1H); 5,5 (s, 1H); 5,15 (m, 1H); 5,0 (t, 1H); 3,45 (m, 2H); 2,80 (d, 3H); 2,3-2,1 (m, 4H); 2,05-1,8 (m, 2H); 1,75-1,5 (m, 2H); 1,4 (s, 3H).

Le tableau 1 qui suit illustre les structures chimiques et les propriétés physiques de quelques composés de formule (I) selon l'invention. Dans ce tableau :
- l'astérisque « * » indique, dans le cas où R₁ et R₂ forment ensemble un cycle (hétérocycle ou cycloalkyle), le carbone d'attache dudit cycle au carbone adjacent de la liaison acétylénique,
- Me et Et représentent respectivement des groupes méthyle et éthyle,
- dans la colonne « sel », « - » représente un composé sous forme de base libre, alors que « HCl » représente un composé sous forme de chlorhydrate,
- la colonne PF indique le point de fusion, en °C, du composé, et
- dans la colonne CUSM, sont indiqués successivement, la méthode analytique de chromatographie liquide haute performance utilisée (A, B ou C) et détaillée ci-dessous, le temps de rétention du composé exprimé en minute, et le pic MH⁺ identifié par spectrométrie de masse.
   - Méthode A :
      Colonne : Kromasil, 50x2,1 mm, 3,5 µm
      Solvant A : H₂O/ACN/TFA (1000/30/0.5); solvant B : ACN/TFA (1000/0.5); débit = 0,5 mL/mn
      Gradient : 100/0 (0 min) to 0/100 (12 min) to 0/100 (15 min)
      Détection : 220 nM
      Ionisation ESI+
   - Méthode B :
      Colonne : Gemini, 50x3 mm, 3µm
      Solvant A : H₂O + 0.1% HCO₂H solvant B : ACN + 0.1% HCO₂H débit = 1 mL/mn Gradient : 95/5 (0 min) to 0/100 (5,5 min) to 0/100 (7,5 min)
      Détection : 220 nM
      Ionisation : ESI+
   - Méthode C :
      Colonne : Kromasil, 50x2,1 mm, 3,5 µm
      Solvant A : CH3CO2NH4 5 mM; solvant B : ACN; débit = 0,5 mL/mn Gradient : 100/0 (0 min) to 0/100 (13 min) to 0/100 (16 min)
      Détection : 220 nM
      Ionisation : ESI+
- dans la colonne CL chirale, sont indiqués successivement pour les composés optiquement purs (pureté énantiomérique > 95%), la méthode analytique de chromatographie liquide haute performance utilisée (D ou E) et détaillée ci-dessous, le temps de rétention du composé exprimé en minute.
   . méthode D :
      Colonne : Chiralpak AD-H, 250x4,6 mm, 5 µM
      Solvant A : 2-propanol/TFA (1000/1); solvant B : heptane/2-propanol (1000/30) Isocratique : 50% A + 50% B
      Débit : 0.8 mL/mn
      Détection : 220 nM
   . méthode E :
      Colonne : Chiralpak AD-H, 250x4,6 mm, 5 µM
      Solvant A : 2-propanol/TFA (1000/1); solvant B : heptane/2-propanol (1000/30) Isocratique : 20% A + 80% B
      Débit : 0,8 mL/mn
      Détection : 220 nM
- dans la colonne chiralité, « / » représente un composé achiral, (±) représente un composé sous forme de mélange racémique, (-) représente un composé sous forme de l'énantiomère lévogyre optiquement pur (pureté énantiomérique > 95%) et (+) représente un composé sous forme de l'énantiomère dextrogyre optiquement pur (pureté énantiomérique > 95%). Dans le cas d'un composé optiquement pur, la valeur du pouvoir rotatoire mesuré à 20°C est indiquée avec entre parenthèses, la concentration c du composé et le solvant utilisé.

Pour obtenir les énantiomères optiquement purs, le mélange racémique correspondant est soumis à une chromatographie préparative sur phase stationnaire chirale (colonne Chiralpak AD-H, 250x21 mm, 5 mm) en utilisant comme phase mobile :
- soit CO₂/2-propanol(70%/30%), avec un débit de 60 mUmn sous une pression de 100 bar
- soit un mélange isohexane/éthanol(70/30) avec 0,3% de TFA et un débit de 120 mL/min

Après élution et évaporation, on isole chaque énantiomère dont la pureté chimique et la pureté énantiomérique sont déterminées par les méthodes analytiques connues de l'Homme de l'art.

**Tableau 1**

| | | | | | | | | | |
|---|---|---|---|---|---|---|---|---|---|
| 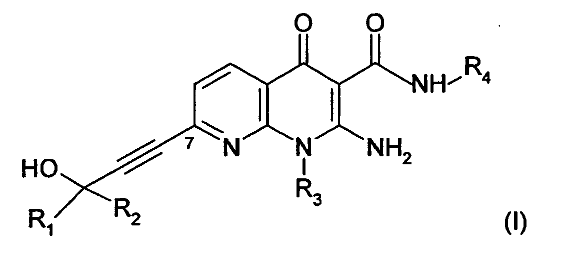 | | | | | | | | | |

| **N°** | **R1** | **R2** | **R3** | **R4** | **Sel** | **CL/ SM** | **CL chirale** | **PF** | **Chiralité** |
|---|---|---|---|---|---|---|---|---|---|
| **1** | H | H | Et | Me | - | B 5.0 301 | - | 278 | / |
| **2** | Me | Me | Me | Me | - | C 6.3 329 | - | 256 | / |
| **3** | | 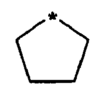 | Et | Me | - | C 6.8 355 | - | 277-279 | (±) |
| **4** | H | Me | Et | Me | - | B 5.4 315 | - | 230 | (±) |
| **5** | Me | Et | Et | Me | - | B 6.1 343 | - | 250 | (±) |
| **6** | | 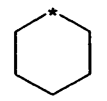 | Et | Me | - | B 4.1 369 | - | 272 | / |
| **7** | | 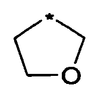 | Et | Me | - | B 5.2 357 | - | 256 | (±) |
| **8** | Me | 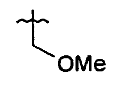 | Et | Me | - | B 5.5 359 | - | 211 | (±) |
| **9** | | 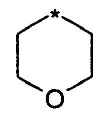 | Et | Me | - | C 5.9 371 | - | 275 | / |
| **10** | H | 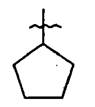 | Et | Me | - | C 7.7 369 | - | 253 | (±) |
| **11** | H | 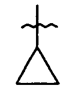 | Et | Me | - | B 5.8 341 | - | 245 | (±) |
| **12** | | 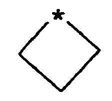 | Et | Me | - | C 6.45 341 | - | 218 | / |
| **13** | Me | 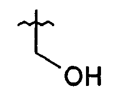 | Et | Me | - | B 4.6 345 | - | 207 | (±) |
| **14** | 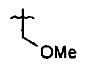 | 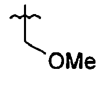 | Et | Me | - | B 3.5 389 | - | 199 | / |
| **15** | Me | 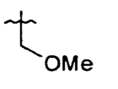 | 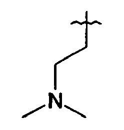 | Me | - | B 3.03 402 | - | 159 | (±) |
| **16** | Me | 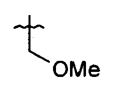 | 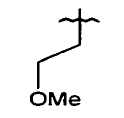 | Me | - | C 6.3 389 | - | 159 | (±) |
| **17** | Me | 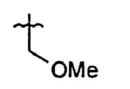 | 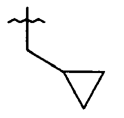 | Me | - | C 6.7 385 | - | 170 | (±) |
| **18** | Me | 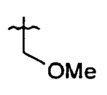 | Me | Me | - | C 5.7 345 | - | 218-219 | (±) |
| **19** | Me | 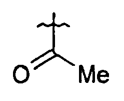 | Et | Me | - | B 3.7 357 | - | 226 | (±) |
| **20** | Me | 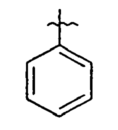 | Et | Me | - | C 7.6 391 | - | 234 | (±) |
| **21** | Me | 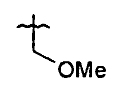 | 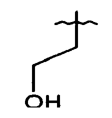 | Me | - | C 5.5 375 | - | 230 | (±) |
| **22** | Me | 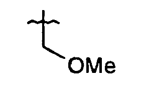 | Et | MeO | - | B 5.2 375 | - | 172 | (±) |
| **23** | Me | 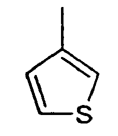 | Et | Me | - | C 7.3 397 | - | 204 | (±) |
| **24** | Me | 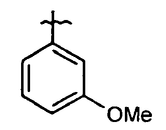 | Et | Me | - | C 7.6 421 | - | 223 | (±) |
| **25** | Me | 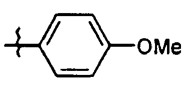 | Et | Me | - | B 11.6 421 | - | 223.5 | (±) |
| **26** | Me | 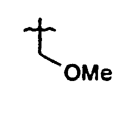 | 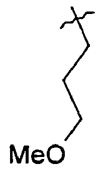 | Me | - | C 6.3 403 | - | 140.5 | (±) |
| **27** | Me | 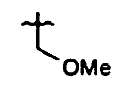 | 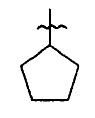 | Me | - | C 7.05 399 | - | 180 | (±) |
| **28** | Me | 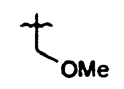 | 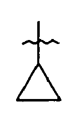 | Me | - | C 5.9 371 | - | 175.5 | (±) |
| **29** | Me | 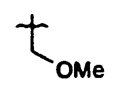 | 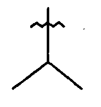 | Me | - | C 6.1 373 | - | 159.5 | (±) |
| **30** | Me | 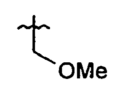 | 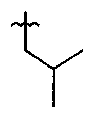 | Me | - | C 6.7 387 | - | 195 | (±) |
| **31** | Me | Me | 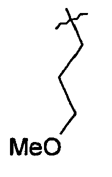 | Me | - | C 6.45 373 | - | 183 | / |
| **32** | 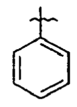 | 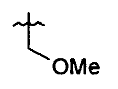 | Et | Me | - | C 7.2 421 | - | 226 | (±) |
| **33** | Me | 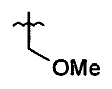 | 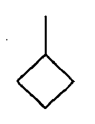 | Me | - | C 6.1 385 | - | 169-170 | (±) |
| **34** | Me | 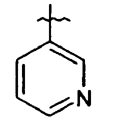 | Et | Me | HCl | C 5.8 392 | - | 150-155 | (±) |
| **35** | Me | 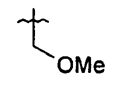 | Et | H | | C 5.0 345 | - | 214 | (±) |
| **36** | Me | 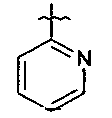 | Et | Me | HCl | C 6.1 429 | - | 292-293 | (±) |
| **37** | Me | 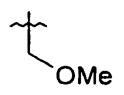 | 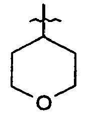 | Me | - | B 5.4 415 | - | 301 | (±) |
| **38** | Me | 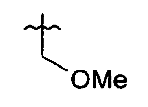 | 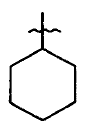 | Me | - | C 6.9 413 | - | 284 | (±) |
| **39** | Me | 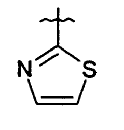 | Et | Me | - | C 6.5 398 | - | 220.4 | (±) |
| **40** | Et | 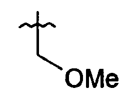 | Et | Me | - | B 5.8 373 | - | 181.5 | (±) |
| **41** | Me | 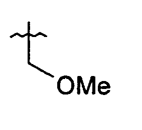 | 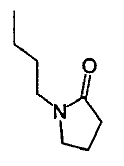 | Me | - | C 5.9 456 | - | 181 | (±) |
| **42** | Me | 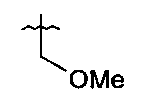 | 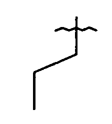 | Me | - | B 6.14 373 | - | 175 | (±) |
| **43** | Me | 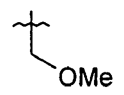 | 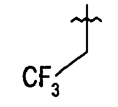 | Me | - | C 6.8 413 | - | 222.5 | (±) |
| **44** | Me | 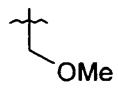 | 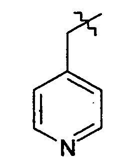 | Me | HCl | B 4.7 422 | - | 232 | (±) |
| **45** | Me | 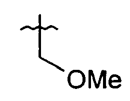 | 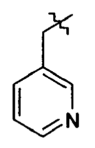 | Me | HCl | C 6.0 422 | - | > 180 | (±) |
| **46** | Me | Me | 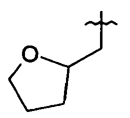 | Me | - | C 6.3 385 | - | 221 | (±) |
| **47** | Me | 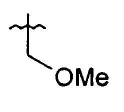 | 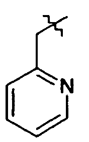 | Me | - | B 5.8 422 | - | 169 | (±) |
| **48** | Me | 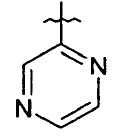 | Et | Me | - | C 5.4 393 | - | 271 | (±) |
| **49** | 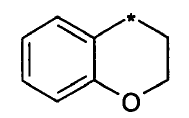 | | Et | Me | - | C 7.2 419 | - | 246.5 | (±) |
| **50** | Me | 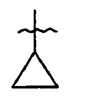 | Et | Me | - | C 6.5 385 | - | 208 | (±) |
| **51** | Me | 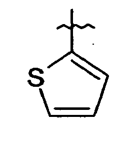 | Et | Me | - | C 7.2 397 | - | 207.5 | (±) |
| **52** | Me | 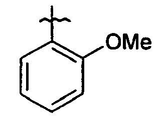 | Et | Me | - | C 7.45 421 | - | 223 | (±) |
| **53** | Me | 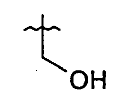 | 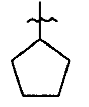 | Me | - | C 6.0 385 | - | 210.5 | (±) |
| **54** | Me | 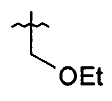 | Et | Me | - | C 6.6 373 | - | 190 | (±) |
| **55** | Me | 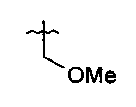 | 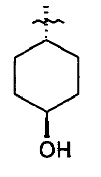 | Me | - | C 5.6 429 | - | 236.5 | (±) |
| **56** | Me | 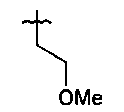 | Et | Me | - | B 6.0 373 | - | - | (±) |
| **57** | Me | 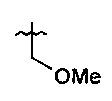 | 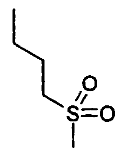 | Me | - | C 6.0 451 | - | 194 | (±) |
| **58** | Me | 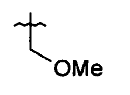 | 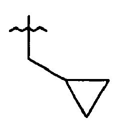 | Me | - | A 6.6 385 | D 5.6 | - | (±) [α]_{D}=-7,2° (c=0,98; MeOH) |
| **59** | Me | 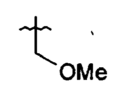 | 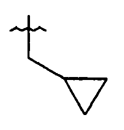 | Me | - | A 6.8 385 | D 6.7 | - | (+) [α]_{D}=+6,1° (c = 0,99; DMSO) |
| **60** | Me | 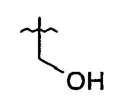 | Et | Me | - | A 4.8 345 | D 12.1 | - | (+) [α]_{D}=+5,1° (c=1; DMSO) |
| **61** | Me | 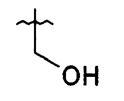 | Et | Me | - | A 4.8 345 | D 9.1 | - | (-) [α]_{D}=-4,2° (c=0,90; DMSO) |
| **62** | Me | 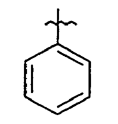 | Et | Me | - | A 7.6 391 | E 13.7 | - | (-) [α]_{D}=-6,4° (c=0,1 ; DMSO) |
| **63** | Me | 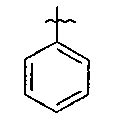 | Et | Me | - | A 7.6 391 | E 24.7 | - | (+) [α]_{D}=+6,7° (c=1; DMSO) |
| **64** | Me | 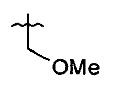 | 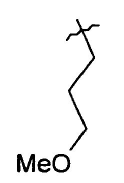 | Me | - | A 6.1 403 | E 14.1 | - | (-) [α]_{D}=-5,0° (c=1; DMSO) |
| **65** | Me | 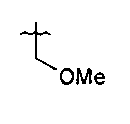 | 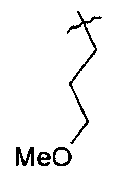 | Me | - | A 6.1 403 | E 18.2 | - | (+) [α]_{D}=+4,9° (c=1; DMSO) |
| **66** | Me | 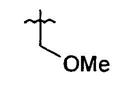 | 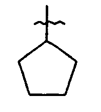 | Me | - | A 7.0 399 | E 11.1 | - | (-) [α]_{D}=-3,6° (c = 0,27; DMSO) |
| **67** | Me | 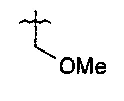 | 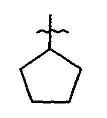 | Me | - | A 7.0 399 | E 15.6 | - | (+) [α]_{D}=+4,9° (c=1; DMSO) |
| **68** | Me | 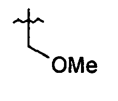 | 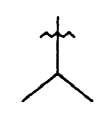 | Me | - | A 6.3 373 | E 30.9 | - | (-) [α]_{D}=-4,2° (c=0,63; DMSO) |
| **69** | Me | 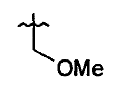 | 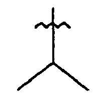 | Me | - | A 6.3 373 | E 12.3 | - | (+) [α]_{D}=+5,3° (c=0,81; |
| **70** | Me | 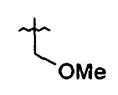 | 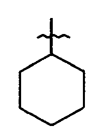 | Me | - | A 7.4 413 | E 12.2 | - | DMSO) (-) [α]_{D}=-4,3° (c=0,85; DMSO) |
| **71** | Me | 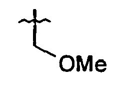 | 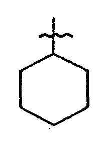 | Me | - | A 7.4 413 | E 19.3 | - | (+) [α]_{D}=+2,5° (c = 0,99; DMSO) |
| **72** | Me | 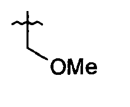 | Et | Me | - | C 6.1 359 | E 5.4 | - | (-) [α]_{D}=-5,1° (c=1; MeOH) |
| **73** | Me | 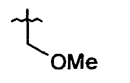 | Et | Me | - | C 6.1 359 | E 6.7 | - | (+) [α]_{D}=+5,4° (c=1, MeOH) |
| **74** | Me | 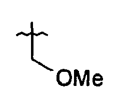 | 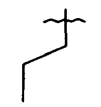 | Me | - | A 6.4 373 | E 12.0 | - | (-) [α]_{D}=-4,3° (c = 0,85; DMSO) |
| **75** | Me | 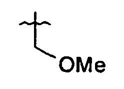 | 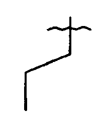 | Me | - | A 6.35 373 | E 31.1 | - | (+) [α]_{D}=+2,9° (c=0,98; DMSO) |
| **76** | Me | 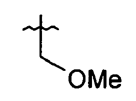 | 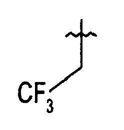 | Me | - | A 6.5 413 | E 11.2 | - | (-) [α]_{D}=-2,4° (c=1; DMSO) |
| **77** | Me | 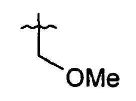 | 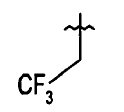 | Me | - | A 6.5 413 | E 31.5 | - | (+) [α]_{D}=+2,6° (c=1,01; DMSO) |
| **78** | Et | 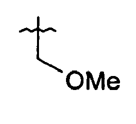 | Et | Me | - | A 6.5 373 | E 12.4 | - | (+) [α]_{D}=+6,5° (c=1,01; DMSO) |
| **79** | Et | 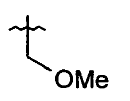 | Et | Me | - | A 6.4 373 | E 21.0 | - | (-) [α]_{D}=-8,7° (c = 0,90; DMSO) |
| **80** | Me | 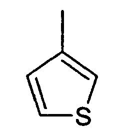 | Et | Me | - | A 7.25 397 | E 15.5 | - | (+) [α]_{D}=+1,9° (c = 0,40; DMSO) |
| **81** | Me | 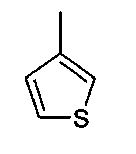 | Et | Me | - | A 7.3 397 | E 25.9 | - | (-) [α]_{D}=-1,8° (c=1; DMSO) |

Les composés selon l'invention ont fait l'objet d'essais pharmacologiques permettant de déterminer leur effet inhibiteur de l'enzyme VEGFR-3.

### Mesure de l'activité tyrosine kinase du VEGFR-3 par ELISA

L'activité enzymatique de VEGFR-3 est évaluée sur un test ELISA par mesure de l'intensité de phosphorylation du substrat poly Glu-Tyr. L'effet des produits est quantifié par la concentration qui diminue l'activité totale de l'enzyme de 50% (IC50). Pour la détermination des IC50, le produit est dilué dans du DMSO avec une gamme de concentration qui s'étale de 3 à 1000 nM. La veille de la manipulation, 125 µl du substrat poly Glu-Tyr (250 µg/ml en PBS 1x sans Ca²⁺ ni Mg²⁺ ni sodium bicarbonate), sont déposés dans chaque puit d'une plaque ELISA (par exemple une plaque ELISA du kit SIGMA Protein Tyrosine Kinase Assay, Ref. PTK-101). La plaque est ensuite recouverte par un adhésif et incubée une nuit à 37°C. Le lendemain, les puits sont vidés par retournement, lavés par ajout de 300 µl de solution tampon (PBS + 0.05% Tween 20) et séchés par une nouvelle incubation de la plaque pendant 2h à 37°C. Un mélange réactionnel de 90 µl est déposé sur chaque puits. Ce mélange contient le tampon kinase 1X additionné de 30 µM d'ATP et de l'inhibiteur à la concentration souhaitée. Ensuite, 20 µl de VEGFR-3-TK (Cell signaling, Ref. 7790), préalablement diluée dans le tampon kinase sans ATP, seront additionnés (à l'exception des puits contrôle négatif où 20 µl de tampon sans enzyme seront rajoutés). Les plaques sont ensuite incubées sous agitation douce à température ambiante pendant 30 min. Après 3 rinçages avec la solution tampon (300µl / puits par lavage), 100 µl d'anticorps anti-Phospho tyrosine-HRP (1 / 30 000). Sont ajoutés à chaque puits et de nouveau les plaques sont Incubées pendant 30 min à température ambiante sous agitation douce. Après 3 lavages en solution tampon (300µl / puits par lavage), la phosphorylation du substrat est révélée par l'addition de 100 µl par puits de substrat OPD, 1 pastille OPD et 1 pastille urée dans 20 ml d'eau (préparation extemporanée et à l'abri de la lumière). Après incubation de 7 minutes à température ambiante et à l'abri de la lumière la réaction est arrêtée par ajout de 100 µl H₂SO₄ à 1,25 M (2,5N) par puit et l'absorbance est lue à 492 nm. L'activité totale est évaluée par la différence de densité optique obtenue sur des échantillons incubés en présence (stimulé) et en absence (non-stimulé) de VEGFR-3.

Les composés conformes à l'invention présentent des Cl50 inférieures à 10 µM, pour la plupart inférieures à 1 µM. A titre d'exemples, les Cl50 de quelques composés du tableau 1 sont indiquées dans le tableau 2 ci-dessous.

**Tableau 2**

| N° du composé (tableau 1) | Cl50 (nM) |
|---|---|
| 2 | 23 |
| 3 | 46 |
| 11 | 37 |
| 12 | 21 |
| 16 | 257 |
| 24 | 6 |
| 37 | 34 |
| 46 | 40 |
| 47 | 65 |
| 60 | 43 |
| 62 | 14 |
| 70 | 5 |
| 72 | 23 |
| 80 | 2 |
| 81 | 38 |

Il apparaît donc que les composés selon l'invention ont une activité inhibitrice de l'enzyme VEGFR-3 ; ils peuvent donc être utilisés pour la préparation de médicaments, en particulier de médicaments inhibiteurs de VEGFR-3.

Ainsi, selon un autre de ses aspects, l'invention a pour objet des médicaments qui comprennent un composé de formule (I), ou un sel d'addition de ce dernier à un acide ou une base pharmaceutiquement acceptable, ou encore un hydrate ou un solvat ainsi qu'un énantiomère ou un diastéréoisomère y compris leur mélange du composé de formule (I).

Un autre aspect de l'invention comprend une association entre au moins un composé selon l'invention et au moins un agent de chimiothérapie.

En effet, les composés de la présente invention peuvent être utilisés seuls ou en mélange avec au moins un agent de chimiothérapie pouvant être choisi parmi :
- les agents alkylants,
- les agents intercalants,
- les agents antimicrotubules,
- les agents antimitotiques,
- les agents antimétabolites,
- les agents anti-prolifératifs,
- les agents antibiotiques,
- les agents immunomodulateurs,
- les agents anti-inflammatoires,
- les inhibiteurs de kinases,
- les agents anti-angiogèniques,
- les agents antivasculaires,
- les hormones oestrogéniques et androgéniques,
et les prodrogues des agents ou dérivés mentionnés ci-dessus.

Il est également possible de combiner les composés selon l'invention avec un traitement par radiations.

Les combinaisons des composés de l'invention avec les agents de chimiothérapie cités ci-dessus et/ou les radiations sont un autre objet de la présente invention.

Les agents de chimiothérapie cités ci-dessus et/ou les radiations peuvent être administrés simultanément, séparément ou séquentiellement. Le traitement sera adapté par le praticien en fonction du malade à traiter.

Ces médicaments trouvent leur emploi en thérapeutique, notamment dans le traitement et/ou la prévention :
- des cancers et de leurs métastases, tels que : glioblastomes, myélomes multiples, syndromes myélodysplasiques, sarcomes de Kaposi, angiosarcomes cutanés, tumeurs solides, lymphomes, mélanomes, cancers du sein, cancers colorectaux, cancers des poumons y compris les cancers non à petites cellules, cancers du pancréas, cancers de la prostate, cancers rénaux, cancers de la tête et du cou, cancer du foie, cancers des ovaires, cancers de l'appareil respiratoire et thoracique, autres tumeurs exprimant VEGFR-3 ou impliquant un processus d'angiogenèse ou de lymphangiogenèse,
- des maladies prolifératives non oncologiques et des angiogenèses pathologiques liées au VEGFR-3, telles que : arthroses, resténoses, psoriasis, hémangiomes, lymphangiomes, glaucomes, glomérulonéphrites, néphropathies diabétiques, néphroscléroses, syndromes microangiopathiques thrombotiques, cirrhoses du foie, athéroscléroses, rejets de greffe d'organe, des maladies de l'oeil impliquant un processus d'angiogénèse ou de lymphangiogenèse telles que la rétinopathie diabétique ou la dégénérescence maculaire,

- ou encore dans le traitement et la prévention de l'inflammation (chronique ou non), de l'infection par les microorganismes et des maladies auto-immunes, telle que la polyarthrite rhumatoïde,
- ou encore dans le traitement de maladies rares telles que la lymphangioléiomyomatose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour le traitement ou la prévention des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) 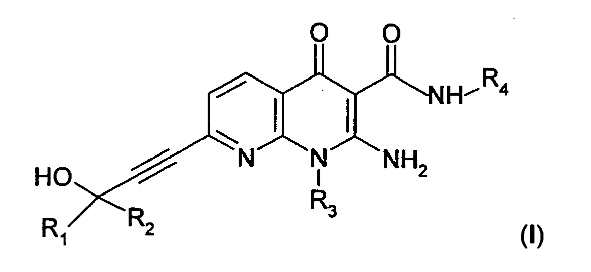 dans laquelle:
R₁ et R₂
**(1)** représentent indépendamment l'un de l'autre:
. soit un atome d'hydrogène,
. soit un groupe alkyle en C1-C7, un groupe -CO-alkyle en C1-C7 ou un groupe cycloalkyle en C3-C8, où lesdits groupes alkyle et cycloalkyle sont éventuellement substitués par un ou plusieurs groupes choisis parmi les atomes d'halogène et les groupes hydroxy et alcoxy,
. soit un groupe phényle éventuellement substitué par un ou plusieurs groupes choisis parmi les atomes d'halogène, les groupes alkyles en C1-C4, alcoxy, en C1-C4, hydroxy, halogénoalcoxy, halogénoalkyle, -CN, -NRR', où R et R' sont tels que définis ci-dessous,
. soit un groupe hétéroaryle éventuellement substitué en des positions quelconques, y compris sur un atome d'azote dudit hétéroaryle, par un ou plusieurs groupes choisis parmi les atomes d'halogène, les groupes alkyles en C1-C4 et les groupes -NRR' où R et R' sont tels que définis ci-dessous,
(**2**) ou R₁ et R₂ forment ensemble, avec l'atome de carbone qui les porte:
. soit un groupe cycloalkyle en C4-C8 ;
. soit un groupe hétérocyclique saturé de 4 à 8 chaînons comprenant un hétéroatome choisi parmi les atomes N, O et S,
et ledit groupe hétérocyclique pouvant être fusionné avec un groupe phényle ;
R₃ représente :
. soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les atomes d'halogène et les groupes hydroxy, alcoxy, -NRR', halogénoalkyle et -SO₂-(C1-C4)alkyl, où R et R' sont tels que définis ci-après ;
. soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O et S, où ledit groupe hétérocyclique est éventuellement substitué par un groupe oxo,
. soit un groupe -(CH₂)ₙ-hétéroaryle, où n = 0, 1, 2 ou 3 et où le groupe hétéroaryle comprend 5 ou 6 chaînons et comprend un ou plusieurs hétéroatomes choisis parmi l'azote, l'oxygène et le soufre ;
R₄ représente un atome d'hydrogène, un groupe alkyle en C1-C4 ou un groupe alcoxy en C1-C4 ;
R et R' représentent indépendamment l'un de l'autre un atome d'hydrogène, un groupe alkyle en C1-C4
à l'état de base ou de sel d'addition à un acide, ainsi que ses énantiomères, diastéréoisomères, ainsi que leur mélange.

2. Composé de formule (I) selon la revendication 1, **caractérisé en ce que**
R₁ et R₂
**(1)** représentent indépendamment l'un de l'autre:
. soit un atome d'hydrogène,
. soit un groupe alkyle en C1-C7, un groupe -CO-alkyle en C1-C7 ou un groupe cycloalkyle en C3-C8, où ledit groupe alkyle est éventuellement substitué par un ou plusieurs groupes choisis parmi les groupes hydroxy et alcoxy,
. oit un groupe phényle éventuellement substitué par un ou plusieurs groupes choisis parmi les groupes alcoxy en C1-C4,
. soit un groupe hétéroaryle,
**(2)** ou R₁ et R₂ forment ensemble, avec l'atome de carbone qui les porte:
. soit un groupe cycloalkyle en C4-C8 ;
. soit un groupe hétérocyclique saturé de 4 à 8 chaînons comprenant un hétéroatome choisi parmi les atomes N, O et S,
et ledit groupe hétérocyclique pouvant être fusionné avec un groupe phényle ;
R₁ représente :
. soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, -NRR', halogénoalkyle et -SO₂-(C1-C4)alkyl, où R et R' sont tels que définis ci-après :
. soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N et O, où ledit groupe hétérocyclique est éventuellement substitué par un groupe oxo,
. soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
R₄ représente un groupe alkyle en C1-C4 ou un groupe alcoxy en C1-C4 ;
R et R' représentent chacun un groupe alkyle en C1-C4 linéaire ou ramifié.
à l'état de base ou de sel d'addition à un acide, ainsi que ses énantiomères, diastéréoisomères, ainsi que leur mélange.

3. Composé de formule (I) selon la revendication 1, **caractérisé en ce que**
R₁ et R₂
(1) représentent indépendamment l'un de l'autre:
. soit un atome d'hydrogène,
. soit un groupe alkyle en C1-C7, un groupe -CO-alkyle en C1-C7 ou un groupe cycloalkyle en C3-C8, où ledit groupe alkyle est éventuellement substitué par un ou plusieurs groupes choisis parmi les groupes hydroxy et alcoxy,
. soit un groupe phényle éventuellement substitué par un ou plusieurs groupes alcoxy en C1-C4,
. soit un groupe hétéroaryle,
(2) ou R₁ et R₂ forment ensemble, avec l'atome de carbone qui les porte:
. soit un groupe cycloalkyle en C4-C8 ;
. soit un groupe hétérocyclique saturé de 4 à 8 chaînons comprenant un hétéroatome d'oxygène,
et ledit groupe hétérocyclique pouvant être fusionné avec un groupe phényle ;
R₃ représente :
. soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, -NRR', halogénoalkyle et -SO2-(C1-C4)alkyl, où R et R' sont tels que définis ci-après ;
. soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O, où ledit groupe hétérocyclique est éventuellement substitué par un groupe oxo,
. soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
R₄ représente un groupe alkyle en C1-C4 ou un groupe alcoxy en C1-C4 ;
R et R' représentent chacun un groupe alkyle en C1-C4 linéaire ou ramifié,
à l'état de base ou de sel d'addition à un acide, ainsi que ses énantiomères, diastéréoisomères, ainsi que leur mélange.

4. Composé de formule (I) selon la revendication 1, **caractérisé en ce que**
R₁ et R₂
(1) représentent indépendamment l'un de l'autre:
. soit un atome d'hydrogène,
. soit un groupe alkyle en C1-C7, ou un groupe cycloalkyle en C3-C8, où ledit groupe alkyle est éventuellement substitué par un ou plusieurs groupes choisis parmi les groupes hydroxy et alcoxy,
. soit un groupe phényle éventuellement substitué par un ou plusieurs groupes alcoxy en C1-C4,
. soit un groupe hétéroaryle,
(2) ou R₁ et R₂ forment ensemble, avec l'atome de carbone qui les porte un groupe cycloalkyle en C4-C8 ;
R₃ représente :
. soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, halogénoalkyle et -SO2-(C1-C4)alkyl;
. soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N, O, où ledit groupe hétérocyclique est éventuellement substitué par un groupe oxo,
. soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
R₄ représente un groupe alkyle en C1-C4 ;
R et R' représentent chacun un groupe alkyle en C1-C4 linéaire ou ramifié ;
à l'état de base ou de sel d'addition à un acide, ainsi que ses énantiomères, diastéréoisomères, ainsi que leur mélange.

5. Composé de formule (I) selon la revendication 1, **caractérisé en ce que** R₁ et R₂
**(1)** représentent indépendamment l'un de l'autre:
. soit un atome d'hydrogène,
. soit un groupe alkyle en C1-C7, un groupe -CO-alkyle en C1-C7 ou un groupe cycloalkyle en C3-C8, où ledit groupe alkyle est éventuellement substitué par un ou plusieurs groupes choisis parmi les groupes hydroxy et alcoxy,
. soit un groupe phényle éventuellement substitué par un ou plusieurs groupes alcoxy en C1-C4,
. soit un groupe hétéroaryle,
(**2**) ou R₁ et R₂ forment ensemble, avec l'atome de carbone qui les porte:
. soit un groupe cycloalkyle en C4-C8 ;
. soit un groupe hétérocyclique saturé de 4 à 8 chaînons comprenant un hétéroatome choisi parmi les atomes N, O et S,
et ledit groupe hétérocyclique pouvant être fusionné avec un groupe phényle ;
à l'état de base ou de sel d'addition à un acide, ainsi que ses énantiomères, diastéréoisomères, ainsi que leur mélange.

6. Composé de formule (I) selon la revendication 1, **caractérisé en ce que** R₃ représente :
. soit un groupe alkyle en C1-C7 linéaire ou ramifié ou un groupe alkyle en C3-C7 dont au moins 3 atomes de carbone sont cyclisés, ledit groupe alkyle étant éventuellement substitué par un ou plusieurs groupes choisis parmi les groupes hydroxy, alcoxy, -NRR' et halogénoalkyle, où R et R' sont tels que définis ci-après ;
. soit un groupe -(CH₂)ₙ-hétérocyclique, où n = 0, 1, 2 ou 3 et où le groupe hétérocyclique comprend entre 4 et 8 chaînons et comprend au moins un hétéroatome choisi parmi les atomes N et O, où ledit groupe hétérocyclique est éventuellement substitué par un groupe oxo,
. soit un groupe -(CH₂)ₙ-hétéroaryle, où n = 0 ou 1 et où le groupe hétéroaryle comprend 5 ou 6 chaînons et comprend un ou plusieurs hétéroatomes d'azote ;
à l'état de base ou de sel d'addition à un acide, ainsi que ses énantiomères, diastéréoisomères, ainsi que leur mélange.

7. Composé de formule (I) selon la revendication 1, **caractérisé en ce que** R₄ représente un groupe alkyle en C1-C4 ou un groupe alcoxy en C1-C4 ;
à l'état de base ou de sel d'addition à un acide, ainsi que ses énantiomères, diastéréoisomères, ainsi que leur mélange.

8. Composé de formule (I) selon la revendication 1, **caractérisé en ce que** R et R' représentent chacun un groupe alkyle en C1-C4 linéaire ou ramifié,
à l'état de base ou de sel d'addition à un acide, ainsi que ses énantiomères, diastéréoisomères, ainsi que leur mélange.

9. Composé de formule (I) selon la revendication 1, **caractérisé en ce qu'**il est choisi parmi:
■ 2-amino-1-éthyl-7-(3-hydroxy-3-méthylbut-1-yn-1-yl)-N-méthyl-4-oxo-1,4-dihydro-1,8-naphthyridine-3-carboxamide
■ (±)-2-amino-1-éthyl-7-[(1-hydroxycyclopentyl)éthynyl]-N-méthyl-4-oxo-1,4-dihydro-1,8-naphthyridine-3-carboxamide
■ (±)-2-amino-1-éthyl-7-(3-hydroxybut-1-yn-1-yl)-N-méthyl-4-oxo-1,4-dihydro-1,8-naphthyridine-3-carboxamide
■ (±)-2-amino-1-éthyl-7-(3-hydroxy-3-méthylpent-1-yn-1-yl)-N-méthyl-4-oxo-1,4-dihydro-1,8-naphthyridine-3-carboxamide
■ (±)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-7-(3-cyclopentyl-3-hydroxyprop-1-yn-1-yl)-1-éthyl-N-méthyl-4-oxo-1,4-dihydro-1,8-naphthyridine-3-carboxamide
■ (±)-2-amino-7-(3-cyclopropyl-3-hydroxyprop-1-yn-1-yl)-1-éthyl-N-méthyl-4-oxo-1,4-dihydro-1,8-naphthyridine-3-carboxamide
■ 2-amino-1-éthyl-7-[(1-hydroxycyclobutyl)éthynyl]-N-méthyl-4-oxo-1,4-dihydro-1,8-naphthyridine-3-carboxamide
■ (±)-2-amino-7-(3,4-dihydroxy-3-méthylbut-1-yn-1-yl)-1-éthyl-N-méthyl-4-oxo-1,4-dihydro-1,8-naphthyridine-3-carboxamide
■ 2-amino-1-éthyl-7-[3-hydroxy-4-méthoxy-3-(méthoxyméthyl)but-1-yn-1-yl]-N-méthyl-4-oxo-1,4-dihydro-1,8-naphthyridine-3-carboxamide
■ (±)-2-amino-1-(cyclopropylméthyl)-7-(3-hydroxy-4-methoxy-3-méthylbut-1-yn-1-yl)-N-méthyl-4-oxo-1,4-dihydro-1,8-naphthyridine-3-carboxamide
■ (±)-2-amino-1-éthyl-7-(3-hydroxy-3-phénylbut-1-yn-1-yl)-N-méthyl-4-oxo-1,4-dihydro-1,8-naphthyridine-3-carboxamide
■ (±)-2-amino-1-éthyl-7-[3-hydroxy-3-(3-thiényl)but-1-yn-1-yl]-N-méthyl-4-oxo-1,4-dihydro-1,8-naphthyridine-3-carboxamide
■ (±)-2-amino-1-éthyl-7-[3-hydroxy-3-(3-méthoxyphényl)but-1-yn-1-yl]-N-méthyl-4-oxo-1,4-dihydro-1,8-naphthyridine-3-carboxamide
■ (±)-2-amino-1-éthyl-7-[3-hydroxy-3-(4-méthoxyphényl)but-1-yn-1-yl]-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-1-(3-méthoxypropyl)-N-méthyl-4-oxo-1,4-dihydro-1,8-naphthyridine-3-carboxamide
■ (±)-2-amino-1-cyclopentyl-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-1-isopropyl-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-1-isobutyl-N-méthyl-4-oxo-1,4-dihydro-1,8-naphthyridine-3-carboxamide
■ 2-amino-7-(3-hydroxy-3-méthylbut-1-yn-1-yl)-1-(3-méthoxypropyl)-N-méthyl-4-oxo-1,4-dihydro-1,8-naphthyridine-3-carboxamide
■ (±)-2-amino-1-éthy)-7-(3-hydroxy-4-méthoxy-3-phénylbut-1-yn-1-yl)-N-méthyl-4-oxo-1,4-dihydro-1,8-naphthyridine-3-carboxamide
■ (±)-2-amino-1-(tétrahydropyran-4-yl)-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-1-cyclohexyl-7-(3-hydroxy-4-méthoxy-3-méthylbut-1-yn-1-yl)-N-méthyl-4-oxo-1,4-dihydro-1,8-naphthyridine-3-carboxamide
■ (±)-2-amino-1-éthyl-7-[3-hydroxy-3-(1,3-thiazol-2-yl)but-1-yn-1-yl]-N-méthyl-4-oxo-1,4-dihydro-1,8-naphthyridine-3-carboxamide
■ (±)-2-amino-1-éthyl-7-[3-hydroxy-3-(méthoxyméthyl)pent-1-yn-1-yl]-N-méthyl-4-oxo-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-[3-(2-oxopyrrolidin-1-yl)propyl]-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-propyl-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-(2,2,2-trifluoroéthyl)-1,4-dihydro-1,8-naphthyridine-3-carboxamide
■ (±)-2-amino-7-(3-hydroxy-3-méthylbut-1-yn-1-yl)-N-méthyl-4-oxo-1-(tetrahydrofuran-2-ylméthyl)-1,4-dihydro-1,8-naphthyridine-3-carboxamide
■ (±)-2-amino-7-(3-hydroxy-4-méthoxy-3-méthylbut-1-yn-1-yl)-N-méthyl-4-oxo-1-(pyridin-2-ylméthyl)-1,4-dihydro-1,8-naphthyridine-3-carboxamide
■ (±)-2-amino-1-éthyl-7-(3-hydroxy-3-pyrazin-2-ylbut-1-yn-1-yl)-N-méthyl-4-oxo-1,4-dihydro-1,8-naphthyridine-3-carboxamide
■ (±)-2-amino-7-(3-cyclopropyl-3-hydroxy-4-méthoxybut-1-yn-1-yl)-1-éthyl-N-méthyl-4-oxo-1,4-dihydro-1,8-naphthyridine-3-carboxamide
■ (±)-2-amino-1-éthyl-7-[3-hydroxy-3-(2-thiényl)but-1-yn-1-yl]-N-méthyl-4-oxo-1,4-dihydro-1,8-naphthyridine-3-carboxamide
■ (±)-2-amino-1-éthyl-7-[3-hydroxy-3-(2-méthoxyphényl)but-1-yn-1-yl]-N-méthyl-4-oxo-1,4-dihydro-1,8-naphthyridine-3-carboxamide
■ (±)-2-amino-1-cyclopentyl-7-(3,4-dihydroxy-3-méthylbut-1-yn-1-yl)-N-méthyl-4-oxo-1,4-dihydro-1,8-naphthyridine-3-carboxamide
■ (±)-2-amino-7-(4-éthoxy-3-hydroxy-3-méthylbut-1-yn-1-yl)-1-éthyl-N-méthyl-4-oxo-1,4-dihydro-1,8-naphthyridine-3-carboxamide
■ (±)-2-amino-1-(trans-4-hydroxycyclohexyl)-7-(3-hydroxy-4-méthoxy-3-méthylbutil-1-yn-1-yl)-N-méthyl-4-oxo-1,4-dihydro-1,8-naphthyridine-3-carboxamide (±)-2-amino-7-(3-hydroxy-4-méthoxy-3-méthylbut-1-yn-1-yl)-N-méthyl-1-[3-(méthylsulfonyl)propyl]-4-oxo-1,4-dihydro-1,8-naphthyridine-3-carboxamide
■ (±)-2-amino-1-(cyclopropylméthyl)-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-(cyclopropylméthyl)-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-7-(3,4-dihydroxy-3-méthylbut-1-yn-1-yl)-1-éthyl-N-méthyl-4-oxo-1,4-dihydro-1,8-naphthyridine-3-carboxamide
■ (-)-2-amino-7-(3,4-dihydroxy-3-méthylbut-1-yn-1-yl)-1-éthyl-N-méthyl-4-oxo-1,4-dihydro-1,8-naphthyridine-3-carboxamide
■ (-)-2-amino-1-éthyl-7-(3-hydroxy-3-phénylbut-1-yn-1-yl)-N-méthyl-4-oxo-1,4-dihydro-1,8-naphthyridine-3-carboxamide
■ (+)-2-amino-1-éthyl-7-(3-hydroxy-3-phénylbut-1-yn-1-yl)-N-méthyl-4-oxo-1,4-dihydro-1,8-naphthyridine-3-carboxamide
■ (-)-2-amino-7-(3-hydroxy-4-méthoxy-3-méthylbut-1-yn-1-yl)-1-(3-méthoxypropyl)-N-méthyl-4-oxo-1,4-dihydro-1,8-naphthyridine-3-carboxamide
■ (+)-2-amino-7-(3-hydroxy-4-méthoxy-3-méthylbut-1-yn-1-yl)-1-(3-méthoxypropyl)-N-méthyl-4-oxo-1,4-dihydro-1,8-naphthyridine-3-carboxamide
■ (-)-2-amino-1-cyclopentyl-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-cyclopentyl-7-(3-hydroxy-4-méthoxy-3-méthylbut-1-yn-1-yl)-N-méthyl-4-oxo-1,4-dihydro-1,8-naphthyridine-3-carboxamide
■ (-)-2-amino-7-(3-hydroxy-4-méthoxy-3-méthylbut-1-yn-1-yl)-1-isopropyl-N-méthyl-4-oxo-1,4-dihydro-1,8-naphthyridine-3-carboxamide
■ (+)-2-amino-7-(3-hydroxy-4-méthoxy-3-méthylbut-1-yn-1-yl)-1-isopropyl-N-méthyl-4-oxo-1,4-dihydro-1,8-naphthyridine-3-carboxamide
■ (-)-2-amino-1-cyclohexyl-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-cyclohexyl-7-(3-hydroxy-4-méthoxy-3-méthylbut-1-yn-1-yl)-N-méthyl-4-oxo-1,4-dihydro-1,8-naphthyridine-3-carboxamide
■ (-)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide
■ (+)-2-amino-1-éthyl-7-(3-hydroxy-4-méthoxy-3-méthylbut-1-yn-1-yl)-N-méthyl-4-oxo-1,4-dihydro-1,8-naphthyridine-3-carboxamide
■ (-)-2-amino-7-(3-hydroxy-4-méthoxy-3-méthylbut-1-yn-1-yl)-N-méthyl-4-oxo-1-propyl-1,4-dihydro-1,8-naphthyridine-3-carboxamide
■ (+)-2-amino-7-(3-hydroxy-4-méthoxy-3-méthylbut-1-yn-1-yl)-N-méthyl-4-oxo-1-propyl-1,4-dihydro-1,8-naphthyridine-3-carboxamide
■ (-)-2-amino-7-(3-hydroxy-4-méthoxy-3-méthylbut-1-yn-1-yl)-N-méthyl-4-oxo-1-(2,2,2-trifluoroéthyl)-1,4-dihydro-1,8-naphthyridine-3-carboxamide
■ (+)-2-amino-7-(3-hydroxy-4-méthoxy-3-méthylbut-1-yn-1-yl)-N-méthyl-4-oxo-1-(2,2,2-trifluoroéthyl)-1,4-dihydro-1,8-naphthyridine-3-carboxamide
■ (+)-2-amino-1-éthyl-7-[3-hydroxy-3-(méthoxyméthyl)pent-1-yn-1-yl]-N-méthyl-4-oxo-1,4-dihydro-1,8-naphthyridine-3-carboxamide
■ (-)-2-amino-1-éthyl-7-[3-hydroxy-3-(méthoxyméthyl)pent-1-yn-1-yl]-N-méthyl-4-oxo-1,4-dihydro-1,8-naphthyridine-3-carboxamide
■ (+)-2-amino-1-éthyl-7-[3-hydroxy-3-(3-thiényl)but-1-yn-1-yl]-N-méthyl-4-oxo-1,4-dihydro-1,8-naphthyridine-3-carboxamide
■ (-)-2-amino-1-éthyl-7-[3-hydroxy-3-(3-thiényl)but-1-yn-1-yl]-N-méthyl-4-oxo-1,4-dihydro-1,8-naphthyridine-3-carboxamide

10. Composé de formule (VII) 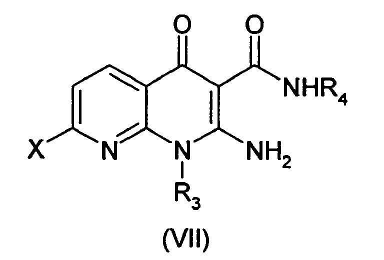 dans laquelle X est un atome d'halogène et R₃ et R₄ sont tels que définis dans l'une des revendications précédentes.

11. Procédé de préparation d'un composé de formule (I) selon l'une quelconque des revendications 1 à 9, **caractérisé en ce que** l'on fait réagir un composé de formule (VII) 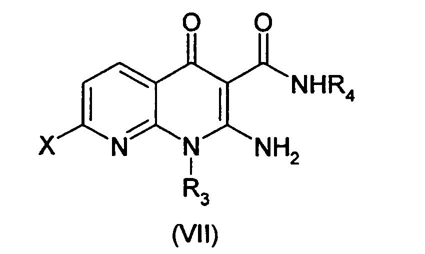 dans laquelle X est un atome d'halogène et R₃ et R₄ sont tels que définis dans l'une des revendications précédentes
avec un composé de formule (VIII) 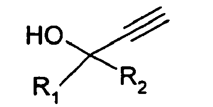 dans laquelle R₁ et R₂ sont tels que définis dans l'une des revendications précédentes.

12. Médicament, **caractérisé en ce qu'**il comprend un composé de formule (I) selon l'une quelconque des revendications 1 à 9, ou un sel d'addition de ce composé à un acide pharmaceutiquement acceptable, ou encore un énantiomère, undiastéréoisomère ou leur mélange du composé de formule (I).

13. Composition pharmaceutique, **caractérisée en ce qu'**elle comprend un composé de formule (I) selon l'une quelconque des revendications 1 à 9, ou un sel pharmaceutiquement acceptable, ou un énantiomère, un diastéréoisomère, leur mélange de ce composé, ainsi qu'au moins un excipient pharmaceutiquement acceptable.

14. Association d'au moins un composé de formule (1) selon l'une quelconque des revendications 1 à 9 avec au moins un agent de chimiothérapie choisi parmi :
• les agents alkylants,
• les agents intercalants,
• les agents antimicrotubules,
• les agents antimitotiques,
• les agents antimétabolites,
• les agents anti-prolifératifs,
• les agents antibiotiques,
• les agents immunomodulateurs,
• les agents anti-inflammatoires,
• les inhibiteurs de kinases,
• les agents anti-angiogèniques,
• les agents antivasculaires,
• les hormones oestrogéniques et androgéniques.

15. Composé de formule (I) selon l'une quelconque des revendications 1 à 9 pour son utilisation pour le traitement et/ou la prévention des cancers et de leurs métastases.

16. Composé de formule (I) selon la revendication 15 pour son utilisation pour le traitement et/ou la prévention des glioblastomes, myélomes multiples, syndromes myélodysplasiques, sarcomes de Kaposi, angiosarcomes cutanés, tumeurs solides, lymphomes, mélanomes, cancers du sein, cancers colorectaux, cancers des poumons y compris les cancers non à petites cellules, cancers du pancréas, cancers de la prostate, cancers rénaux, cancers de la tête et du cou, cancer du foie, cancers des ovaires, cancers de l'appareil respiratoire et thoracique, autres tumeurs exprimant VEGFR-3 ou impliquant un processus d' angiogénèse ou de lymphangiogénèse.

17. Composé de formule (I) pour son utilisation pour la préparation d'un médicament destiné au traitement et/ou à la prévention des maladies choisies parmi arthroses, resténoses, psoriasis, hémangiomes, lymphangiomes, glaucomes, glomérulonéphrites, néphropathies diabétiques, néphroscléroses, syndromes microangiopathiques thrombotiques, cirrhoses du foie, athéroscléroses, rejets de greffe d'organe, maladies de l'oeil impliquant un processus d'angiogénèse ou de lymphangiogenèse.

18. Composé de formule (I) selon l'une quelconque des revendications 1 à 9 pour la préparation d'un médicament destiné au traitement et/ou à la prévention de l'inflammation chronique ou non, de l'infection par les microorganismes et des maladies auto-immunes, telle que la polyarthrite rhumatoïde.

19. Composé de formule (I) selon l'une quelconque des revendications 1 à 9 pour son utilisation pour le traitement et/ou la prévention de maladies rares telles que la lymphangioleiomyomatose.

## Claims

1. Compound corresponding to formula (I) 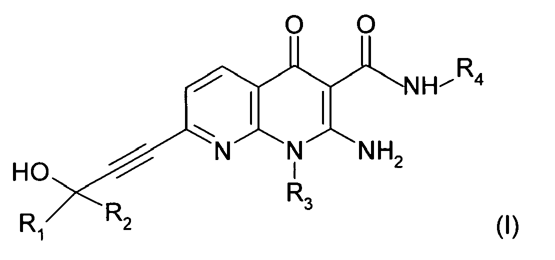 in which:
R₁ and R₂
**(1)** are, independently of one another:
. either a hydrogen atom,
. or a C₁-C₇ alkyl group, a -CO-(C₁-C₇) alkyl group or a C₃-C₈ cycloalkyl group, where said alkyl and cycloalkyl groups are optionally substituted with one or more groups selected from halogen atoms and hydroxyl and alkoxy groups,
. or a phenyl group optionally substituted with one or more groups selected from halogen atoms, and C₁-C₄ alkyl, C₁-C₄ alkoxy, hydroxyl, haloalkoxy, haloalkyl, -CN or -NRR' groups, where R and R' are as defined below,
. or a heteroaryl group optionally substituted in any positions, including on a nitrogen atom of said heteroaryl, with one or more groups selected from halogen atoms, C₁-C₄ alkyl groups and -NRR' groups, where R and R' are as defined below,
**(2)** or R₁ and R₂ form, together with the carbon atom that bears them:
. either a C₄-C₈ cycloalkyl group,
. or a 4- to 8-membered saturated heterocyclic group comprising a heteroatom selected from N, O and S atoms,
and it being possible for said heterocyclic group to be fused with a phenyl group;
R₃ is:
. either a linear or branched C₁-C₇ alkyl group or a C₃-C₇ alkyl group in which at least 3 carbon atoms are cyclized, said alkyl group being optionally substituted with one or more groups selected from halogen atoms and hydroxyl, alkoxy, -NRR', -haloalkyl and -SO₂-(C₁-C₄)alkyl groups, where R and R' are as defined hereinafter,
. or a -(CH₂)ₙ-heterocyclic group, where n = 0, 1, 2 or 3 and where the heterocyclic group comprises between 4 and 8 members and comprises at least one heteroatom selected from N, O and S atoms, where said heterocyclic group is optionally substituted with an oxo group,
. or a -(CH₂)ₙ-heteroaryl group, where n = 0, 1, 2 or 3 and where the heteroaryl group comprises 5 or 6 members and comprises one or more heteroatoms selected from nitrogen, oxygen and sulphur;
R₄ is a hydrogen atom, a C₁-C₄ alkyl group or a C₁-C₄ alkoxy group;
R and R' are, independently of one another, a hydrogen atom or a C₁-C₄ alkyl group,
in the form of a base or of an addition salt with an acid, and also enantiomers and diastereoisomers thereof, and also a mixture thereof.

2. Compound of formula (I) according to Claim 1, **characterized in that**
R₁ and R₂
**(1)** are, independently of one another:
. either a hydrogen atom,
. or a C₁-C₇ alkyl group, a -CO-(C₁-C₇)alkyl group or a C₃-C₈ cycloalkyl group, where said alkyl group is optionally substituted with one or more groups selected from hydroxyl and alkoxy groups,
. or a phenyl group optionally substituted with one or more groups selected from C₁-C₄ alkoxy groups,
. or a heteroaryl group,
**(2)** or R₁ and R₂ form, together with the carbon atom which bears them:
• either a C₄-C₈ cycloalkyl group,
• or a 4- to 8-membered saturated heterocyclic group comprising a heteroatom selected from N, O and S atoms,
and it being possible for said heterocyclic group to be fused with a phenyl group;
R₃ is:
• either a linear or branched C₁-C₇ alkyl group or a C₃-C₇ alkyl group in which at least 3 carbon atoms are cyclized, said alkyl group being optionally substituted with one or more groups selected from hydroxyl, alkoxy, -NRR', halooalkyl and -SO₂-(C₁-C₄)alkyl groups, where R and R' are as defined hereinafter;
• or a -(CH₂)ₙ-heterocyclic group, where n = 0, 1, 2 or 3 and where the heterocyclic group comprises between 4 and 8 members and comprises at least one heteroatom selected from N and O atoms, where said heterocyclic group is optionally substituted with an oxo group,
• or a -(CH₂)ₙ-heteroaryl group, where n = 0 or 1 and where the heteroaryl group comprises 5 or 6 members and comprises one or more nitrogen heteroatoms;
R₄ is a C₁-C₄ alkyl group or a C₁-C₄ alkoxy group;
R and R' are each a linear or branched C₁-C₄ alkyl group,
in the form of a base or of an addition salt with an acid, and also enantiomers and diastereoisomers thereof, and also a mixture thereof.

3. Compound of formula (I) according to Claim 1, **characterized in that**
R₁ and R₂
**(1)** are, independently of one another:
• either a hydrogen atom,
• or a C₁-C₇ alkyl group, a -CO-(C₁-C₇)alkyl group or a C₃-C₈ cycloalkyl group, where said alkyl group is optionally substituted with one or more groups selected from hydroxyl and alkoxy groups,
• or a phenyl group optionally substituted with one or more C₁-C₄ alkoxy groups,
• or a heteroaryl group,
**(2)** or R₁ and R₂ form, together with the carbon atom which bears them:
• either a C₄-C₈ cycloalkyl group,
• or a 4- to 8-membered saturated heterocyclic group comprising an oxygen heteroatom,
and it being possible for said heterocyclic group to be fused with a phenyl group;
R₃ is:
• either a linear or branched C₁-C₇ alkyl group or a C₃-C₇ alkyl group in which at least 3 carbon atoms are cyclized, said alkyl group being optionally substituted with one or more groups selected from hydroxyl, alkoxy, -NRR', haloalkyl and -SO₂-(C₁-C₄) alkyl groups, where R and R' are as defined hereinafter;
• or a -(CH₂)ₙ-heterocyclic group, where n = 0, 1, 2 or 3 and where the heterocyclic group comprises between 4 and 8 members and comprises at least one heteroatom chosen from N and 0 atoms, where said heterocyclic group is optionally substituted with an oxo group,
• or a -(CH₂)ₙ-heteroaryl group, where n = 0 or 1 and where the heteroaryl group comprises 5 or 6 members and comprises one or more nitrogen heteroatoms;
R₄ is a C₁-C₄ alkyl group or a C₁-C₄ alkoxy group;
R and R' are each a linear or branched C₁-C₄ alkyl group,
in the form of a base or an addition salt with an acid, and also enantiomers and diastereoisomers thereof, and also a mixture thereof.

4. Compound of formula (I) according to Claim 1, **characterized in that**
R₁ and R₂
**(1)** are, independently of one another:
• either a hydrogen atom,
• or a C₁-C₇ alkyl group or a C₃-C₈ cycloalkyl group, where said alkyl group is optionally substituted with one or more groups selected from hydroxyl and alkoxy groups,
• or a phenyl group optionally substituted with one or more C₁-C₄ alkoxy groups,
• or a heteroaryl group,
**(2)** or R₁ and R₂ form, together with the carbon atom which bears them, a C₄-C₈ cycloalkyl group;
R₃ is:
. either a linear or branched C₁-C₇ alkyl group or a C₃-C₇ alkyl group in which at least 3 carbon atoms are cyclized, said alkyl group being optionally substituted with one or more groups selected from hydroxyl, alkoxy, haloalkyl and -SO₂-(C₁-C₄) alkyl groups,
. or a -(CH₂)ₙ-heterocyclic group, where n = 0, 1, 2 or 3 and where the heterocyclic group comprises between 4 and 8 members and comprises at least one heteroatom selected from N and 0 atoms, where said heterocyclic group is optionally substituted with an oxo group,
• or a -(CH₂)ₙ-heteroaryl group, where n = 0 or 1 and where the heteroaryl group comprises 5 or 6 members and comprises one or more nitrogen heteroatoms;
R₄ is a C₁-C₄ alkyl group;
R and R' are each a linear or branched C₁-C₄ alkyl group;
in the form of a base or an addition salt with an acid, and also enantiomers and diastereoisomers thereof, and also a mixture thereof.

5. Compound of formula (I) according to Claim 1, **characterized in that** R₁ and R₂
**(1)** represent, independently of one another:
• either a hydrogen atom,
• or a C₁-C₇ alkyl group, a -CO-(C₁-C₇)alkyl group or a C₃-C₈ cycloalkyl group, where said alkyl group is optionally substituted with one or more groups selected from hydroxyl and alkoxy groups,
• or a phenyl group optionally substituted with one or more C₁-C₄ alkoxy groups,
• or a heteroaryl group,
**(2)** or R₁ and R₂ form, together with the carbon atom which bears them:
• either a C₄-C₈ cycloalkyl group,
• or a 4- to 8-membered saturated heterocyclic group comprising a heteroatom selected from N, O and S atoms,
and it being possible for said heterocyclic group to be fused with a phenyl group;
in the form of a base or of an addition salt with an acid, and also enantiomers and diastereoisomers thereof, and also a mixture thereof.

6. Compound of formula (I) according to Claim 1, **characterized in that** R₃ is:
• either a linear or branched C₁-C₇ alkyl group or a C₃-C₇ alkyl group in which at least 3 carbon atoms are cyclized, said alkyl group being optionally substituted with one or more groups selected from hydroxyl, alkoxy, -NRR' and haloalkyl groups, where R and R' are as defined hereinafter;
• or a -(CH₂)ₙ-heterocyclic group, where n = 0, 1, 2 or 3 and where the heterocyclic group comprises between 4 and 8 members and comprises at least one heteroatom chosen from N and O atoms, where said heterocyclic group is optionally substituted with an oxo group,
• or a -(CH₂)ₙ-heteroaryl group, where n = 0 or 1 and where the heteroaryl group comprises 5 or 6 members and comprises one or more nitrogen heteroatoms;
in the form of a base or an addition salt with an acid, and also enantiomers and diastereoisomers thereof, and also a mixture thereof.

7. Compound of formula (I) according to Claim 1, **characterized in that** R₄ is a C₁-C₄ alkyl group or a C₁-C₄ alkoxy group;
in the form of a base or of an addition salt with an acid, and also enantiomers and diastereoisomers thereof, and also a mixture thereof.

8. Compound of formula (I) according to Claim 1, **characterized in that** R and R' are each a linear or branched C₁-C₄ alkyl group,
in the form of a base or of an addition salt with an acid, and also enantiomers and diastereoisomers thereof, and also a mixture thereof.

9. Compound of formula (I) according to Claim 1, **characterized in that** it is selected from:
■ 2-amino-1-ethyl-7-(3-hydroxy-3-methylbut-1-yn-1-yl)-N-methyl-4-oxo-1,4-dihydro-1,8-naphthyridine-3-carboxamide
■ (±)-2-amino-1-ethyl-7-[(1-hydroxycyclopentyl)ethynyl]-N-methyl-4-oxo-1,4-dihydro-1,8-naphthyridine-3-carboxamide
■ (±)-2-amino-1-ethyl-7-(3-hydroxybut-1-yn-1-yl)-N-methyl-4-oxo-1,4-dihydro-1,8-naphthyridine-3-carboxamide
■ (±)-2-amino-1-ethyl-7-(3-hydroxy-3-methylpent-1-yn-1-yl)-N-methyl-4-oxo-1,4-dihydro-1,8-naphthyridine-3-carboxamide
■ (±)-2-amino-1-ethyl-7-(3-hydroxy-4-methoxy-3-methylbut-1-yn-1-yl)-N-methyl-4-oxo-1,4-dihydro-1,8-naphthyridine-3-carboxamide
■ (±)-2-amino-7-(3-cyclopentyl-3-hydroxyprop-1-yn-1-yl)-1-ethyl-N-methyl-4-oxo-1,4-dihydro-1,8-naphthyridine-3-carboxamide
■ (±)-2-amino-7-(3-cyclopropyl-3-hydroxyprop-1-yn-1-yl)-1-ethyl-N-methyl-4-oxo-1,4-dihydro-1,8-naphthyridine-3-carboxamide
■ 2-amino-1-ethyl-7-[(1-hydroxycyclobutyl)ethynyl]-N-methyl-4-oxo-1,4-dihydro-1,8-naphthyridine-3-carboxamide
■ (±)-2-amino-7-(3,4-dihydroxy-3-methylbut-1-yn-1-yl)-1-ethyl-N-methyl-4-oxo-1,4-dihydro-1,8-naphthyridine-3-carboxamide
■ 2-amino-1-ethyl-7-[3-hydroxy-4-methoxy-3-(methoxymethyl)but-1-yn-1-yl]-N-methyl-4-oxo-1,4-dihydro-1,8-naphthyridine-3-carboxamide
■ (±)-2-amino-1-(cyclopropylmethyl)-7-(3-hydroxy-4-methoxy-3-methylbut-1-yn-1-yl)-N-methyl-4-oxo-1,4-dihydro-1,8-naphthyridine-3-carboxamide
■ (±)-2-amino-1-ethyl-7-(3-hydroxy-3-phenylbut-1-yn-1-yl)-N-methyl-4-oxo-1,4-dihydro-1,8-naphthyridine-3-carboxamide
■ (±)-2-amino-1-ethyl-7-[3-hydroxy-3-(3-thienyl)but-1-yn-1-yl]-N-methyl-4-oxo-1,4-dihydro-1,8-naphthyridine-3-carboxamide
■ (±)-2-amino-1-ethyl-7-[3-hydroxy-3-(3-methoxyphenyl)but-1-yn-1-yl]-N-methyl-4-oxo-1,4-dihydro-1,8-naphthyridine-3-carboxamide
■ (±)-2-amino-1-ethyl-7-[3-hydroxy-3-(4-methoxyphenyl)but-1-yn-1-yl]-N-methyl-4-oxo-1,4-dihydro-1,8-naphthyridine-3-carboxamide
■ (±)-2-amino-7-(3-hydroxy-4-methoxy-3-methylbut-1-yn-1-yl)-1-(3-methoxypropyl)-N-methyl-4-oxo-1,4-dihydro-1,8-naphthyridine-3-carboxamide
■ (±)-2-amino-1-cyclopentyl-7-(3-hydroxy-4-methoxy-3-methylbut-1-yn-1-yl)-N-methyl-4-oxo-1,4-dihydro-1,8-naphthyridine-3-carboxamide
■ (±)-2-amino-7-(3-hydroxy-4-methoxy-3-methylbut-1-yn-1-yl)-1-isopropyl-N-methyl-4-oxo-1,4-dihydro-1,8-naphthyridine-3-carboxamide
■ (±)-2-amino-7-(3-hydroxy-4-methoxy-3-methylbut-1-yn-1-yl)-1-isobutyl-N-methyl-4-oxo-1,4-dihydro-1,8-naphthyridine-3-carboxamide
■ 2-amino-7-(3-hydroxy-3-methylbut-1-yn-1-yl)-1-(3-methoxypropyl)-N-methyl-4-oxo-1,4-dihydro-1,8-naphthyridine-3-carboxamide
■ (±)-2-amino-1-ethyl-7-(3-hydroxy-4-methoxy-3-phenylbut-1-yn-1-yl)-N-methyl-4-oxo-1,4-dihydro-1,8-naphthyridine-3-carboxamide
■ (±)-2-amino-1-(tetrahydropyran-4-yl)-7-(3-hydroxy-4-methoxy-3-methylbut-1-yn-1-yl)-N-methyl-4-oxo-1,4-dihydro-1,8-naphthyridine-3-carboxamide
■ (±)-2-amino-1-cyclohexyl-7-(3-hydroxy-4-methoxy-3-methylbut-1-yn-1-yl)-N-methyl-4-oxo-1,4-dihydro-1,8-naphthyridine-3-carboxamide
■ (±)-2-amino-1-ethyl-7-[3-hydroxy-3-(1,3-thiazol-2-yl)but-1-yn-1-yl]-N-methyl-4-oxo-1,4-dihydro-1,8-naphthyridine-3-carboxamide
■ (±)-2-amino-1-ethyl-7-[3-hydroxy-3-(methoxymethyl)pent-1-yn-1-yl]-N-methyl-4-oxo-1,4-dihydro-1,8-naphthyridine-3-carboxamide
■ (±)-2-amino-7-(3-hydroxy-4-methoxy-3-methylbut-1-yn-1-yl)-N-methyl-4-oxo-1-[3-(2-oxopyrrolidin-1-yl)propyl]-1,4-dihydro-1,8-naphthyridine-3-carboxamide
■ (±)-2-amino-7-(3-hydroxy-4-methoxy-3-methylbut-1-yn-1-yl)-N-methyl-4-oxo-1-propyl-1,4-dihydro-1,8-naphthyridine-3-carboxamide
■ (±)-2-amino-7-(3-hydroxy-4-methoxy-3-methylbut-1-yn-1-yl)-N-methyl-4-oxo-1-(2,2,2-trifluoroethyl)-1,4-dihydro-1,8-naphthyridine-3-carboxamide
■ (±)-2-amino-7-(3-hydroxy-3-methylbut-1-yn-1-yl)-N-methyl-4-oxo-1-(tetrahydrofuran-2-ylmethyl)-1,4-dihydro-1,8-naphthyridine-3-carboxamide
■ (±)-2-amino-7-(3-hydroxy-4-methoxy-3-methylbut-1-yn-1-yl)-N-methyl-4-oxo-1-(pyridin-2-ylmethyl)-1,4-dihydro-1,8-naphthyridine-3-carboxamide
■ (±)-2-amino-1-ethyl-7-(3-hydroxy-3-pyrazin-2-ylbut-1-yn-1-yl)-N-methyl-4-oxo-1,4-dihydro-1,8-naphthyridine-3-carboxamide
■ (±)-2-amino-7-(3-cyclopropyl-3-hydroxy-4-methoxybut-1-yn-1-yl)-1-ethyl-N-methyl-4-oxo-1,4-dihydro-1,8-naphthyridine-3-carboxamide
■ (±)-2-amino-1-ethyl-7-[3-hydroxy-3-(2-thienyl)but-1-yn-1-yl]-N-methyl-4-oxo-1,4-dihydro-1,8-naphthyridine-3-carboxamide
■ (±)-2-amino-1-ethyl-7-[3-hydroxy-3-(2-methoxyphenyl)but-1-yn-1-yl]-N-methyl-4-oxo-1,4-dihydro-1,8-naphthyridine-3-carboxamide
■ (±)-2-amino-1-cyclopentyl-7-(3,4-dihydroxy-3-methylbut-1-yn-1-yl)-N-methyl-4-oxo-1,4-dihydro-1,8-naphthyridine-3-carboxamide
■ (±)-2-amino-7-(4-ethoxy-3-hydroxy-3-methylbut-1-yn-1-yl)-1-ethyl-N-methyl-4-oxo-1,4-dihydro-1,8-naphthyridine-3-carboxamide
■ (±)-2-amino-1-(trans-4-hydroxycyclohexyl)-7-(3-hydroxy-4-methoxy-3-methylbut-1-yn-1-yl)-N-methyl-4-oxo-1,4-dihydro-1,8-naphthyridine-3-carboxamide
■ (±)-2-amino-7-(3-hydroxy-4-methoxy-3-methylbut-1-yn-1-yl)-N-methyl-1-[3-(methylsulphonyl)propyl]-4-oxo-1,4-dihydro-1,8-naphthyridine-3-carboxamide
■ (-)-2-amino-1-(cyclopropylmethyl)-7-(3-hydroxy-4-methoxy-3-methylbut-1-yn-1-yl)-N-methyl-4-oxo-1,4-dihydro-1,8-naphthyridine-3-carboxamide
■ (+)-2-amino-1-(cyclopropylmethyl)-7-(3-hydroxy-4-methoxy-3-methylbut-1-yn-1-yl)-N-methyl-4-oxo-1,4-dihydro-1,8-naphthyridine-3-carboxamide
■ (+)-2-amino-7-(3,4-dihydroxy-3-methylbut-1-yn-1-yl)-1-ethyl-N-methyl-4-oxo-1,4-dihydro-1,8-naphthyridine-3-carboxamide
■ (-)-2-amino-7-(3,4-dihydroxy-3-methylbut-1-yn-1-yl)-1-ethyl-N-methyl-4-oxo-1,4-dihydro-1,8-naphthyridine-3-carboxamide
■ (-)-2-amino-1-ethyl-7-(3-hydroxy-3-phenylbut-1-yn-1-yl)-N-methyl-4-oxo-1,4-dihydro-1,8-naphthyridine-3-carboxamide
■ (+)-2-amino-1-ethyl-7-(3-hydroxy-3-phenylbut-1-yn-1-yl)-N-methyl-4-oxo-1,4-dihydro-1,8-naphthyridine-3-carboxamide
■ (-)-2-amino-7-(3-hydroxy-4-methoxy-3-methylbut-1-yn-1-yl)-1-(3-methoxypropyl)-N-methyl-4-oxo-1,4-dihydro-1,8-naphthyridine-3-carboxamide
■ (+)-2-amino-7-(3-hydroxy-4-methoxy-3-methylbut-1-yn-1-yl)-1-(3-methoxypropyl)-N-methyl-4-oxo-1,4-dihydro-1,8-naphthyridine-3-carboxamide
■ (-)-2-amino-1-cyclopentyl-7-(3-hydroxy-4-methoxy-3-methylbut-1-yn-1-yl)-N-methyl-4-oxo-1,4-dihydro-1,8-naphthyridine-3-carboxamide
■ (+)-2-amino-1-cyclopentyl-7-(3-hydroxy-4-methoxy-3-methylbut-1-yn-1-yl)-N-methyl-4-oxo-1,4-dihydro-1,8-naphthyridine-3-carboxamide
■ (-)-2-amino-7-(3-hydroxy-4-methoxy-3-methylbut-1-yn-1-yl)-1-isopropyl-N-methyl-4-oxo-1,4-dihydro-1,8-naphthyridine-3-carboxamide
■ (+)-2-amino-7-(3-hydroxy-4-methoxy-3-methylbut-1-yn-1-yl)-1-isopropyl-N-methyl-4-oxo-1,4-dihydro-1,8-naphthyridine-3-carboxamide
■ (-)-2-amino-1-cyclohexyl-7-(3-hydroxy-4-methoxy-3-methylbut-1-yn-1-yl)-N-methyl-4-oxo-1,4-dihydro-1,8-naphthyridine-3-carboxamide
■ (+)-2-amino-1-cyclohexyl-7-(3-hydroxy-4-methoxy-3-methylbut-1-yn-1-yl)-N-methyl-4-oxo-1,4-dihydro-1,8-naphthyridine-3-carboxamide
■ (-)-2-amino-1-ethyl-7-(3-hydroxy-4-methoxy-3-methylbut-1-yn-1-yl)-N-methyl-4-oxo-1,4-dihydro-1,8-naphthyridine-3-carboxamide
■ (+)-2-amino-1-ethyl-7-(3-hydroxy-4-methoxy-3-methylbut-1-yn-1-yl)-N-methyl-4-oxo-1,4-dihydro-1,8-naphthyridine-3-carboxamide
■ (-)-2-amino-7-(3-hydroxy-4-methoxy-3-methylbut-1-yn-1-yl)-N-methyl-4-oxo-1-propyl-1,4-dihydro-1,8-naphthyridine-3-carboxamide
■ (+)-2-amino-7-(3-hydroxy-4-methoxy-3-methylbut-1-yn-1-yl)-N-methyl-4-oxo-1-propyl-1,4-dihydro-1,8-naphthyridine-3-carboxamide
■ (-)-2-amino-7-(3-hydroxy-4-methoxy-3-methylbut-1-yn-1-yl)-N-methyl-4-oxo-1-(2,2,2-trifluoroethyl)-1,4-dihydro-1,8-naphthyridine-3-carboxamide
■ (+)-2-amino-7-(3-hydroxy-4-methoxy-3-methylbut-1-yn-1-yl)-N-methyl-4-oxo-1-(2,2,2-trifluoroethyl)-1,4-dihydro-1,8-naphthyridine-3-carboxamide
■ (+)-2-amino-1-ethyl-7-[3-hydroxy-3-(methoxymethyl)pent-1-yn-1-yl]-N-methyl-4-oxo-1,4-dihydro-1,8-naphthyridine-3-carboxamide
■ (-)-2-amino-1-ethyl-7-[3-hydroxy-3-(methoxymethyl)pent-1-yn-1-yl]-N-methyl-4-oxo-1,4-dihydro-1,8-naphthyridine-3-carboxamide
■ (+)-2-amino-1-ethyl-7-[3-hydroxy-3-(3-thienyl)but-1-yn-1-yl]-N-methyl-4-oxo-1,4-dihydro-1,8-naphthyridine-3-carboxamide
■ (-)-2-amino-1-ethyl-7-[3-hydroxy-3-(3-thienyl)but-1-yn-1-yl]-N-methyl-4-oxo-1,4-dihydro-1,8-naphthyridine-3-carboxamide.

10. Compound of formula (VII) 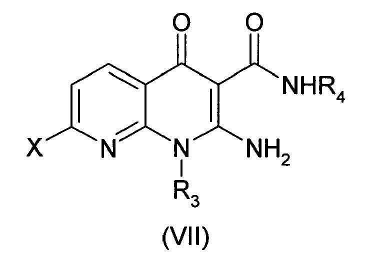 in which X is a halogen atom and R₃ and R₄ are as defined in one of the preceding claims.

11. Process for preparing a compound of formula (I) according to any one of Claims 1 to 9, **characterized in that** a compound of formula (VII) 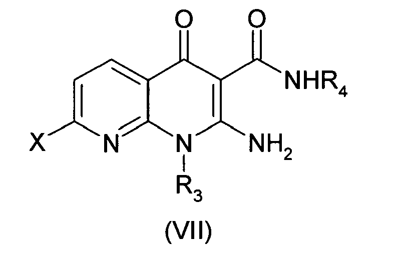 in which X is a halogen atom and R₃ and R₄ are as defined in one of the preceding claims, is reacted with a compound of formula (VIII) 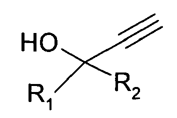 in which R₁ and R₂ are as defined in one of the preceding claims.

12. Medicament, **characterized in that** it comprises a compound of formula (I) according to any one of Claims 1 to 9, or an addition salt of this compound with a pharmaceutically acceptable acid, or else an enantiomer or a diastereoisomer, or a mixture thereof, of the compound of formula (I).

13. Pharmaceutical composition, **characterized in that** it comprises a compound of formula (I) according to any one of Claims 1 to 9, or a pharmaceutically acceptable salt, or an enantiomer or a diastereoisomer, or a mixture thereof, of this compound, and also at least one pharmaceutically acceptable excipient.

14. Combination of at least one compound of formula (I) according to any one of Claims 1 to 9 and at least one chemotherapy agent selected from:
• alkylating agents,
• intercalating agents,
• antimicrotubule agents,
• antimitotics,
• antimetabolites,
• antiproliferative agents,
• antibiotics,
• immunomodulatory agents,
• anti-inflammatories,
• kinase inhibitors,
• anti-angiogenic agents,
• antivascular agents,
• oestrogenic and androgenic hormones.

15. Compound of formula (I) according to any one of Claims 1 to 9, for use in the treatment and/or prevention of cancers and metastases thereof.

16. Compound of formula (I) according to Claim 15, for use in the treatment and/or prevention of glioblastomas, multiple myelomas, myelodysplasic syndromes, Kaposi's sarcomas, cutaneous angiosarcomas, solid tumours, lymphomas, melanomas, breast cancers, colorectal cancers, lung cancers, including non-small-cell cancers, pancreatic cancers, prostate cancers, kidney cancers, head and neck cancers, liver cancer, ovarian cancers, cancers of the respiratory tract and chest, or other tumours expressing VEGFR-3 or involving a process of angiogenesis or of lymphangiogenesis.

17. Compound of formula (I) for use in the preparation of a medicament for the treatment and/or prevention of diseases among arthrosis, restenosis, psoriasis, hemangiomas, lymphangiomas, glaucomas, glomerulonephritis, diabetic nephropathies, nephrosclerosis, thrombotic microangiopathic syndromes, liver cirrhosis, atherosclerosis, organ transplant rejection, or eye diseases involving a process of angiogenesis or of lymphangiogenesis.

18. Compound of formula (I) according to any one of Claims 1 to 9, for the preparation of a medicament for the treatment and/or prevention of chronic or non-chronic inflammation, of infection with microorganisms and of autoimmune diseases, such as rheumatoid arthritis.

19. Compound of formula (I) according to any one of Claims 1 to 9, for use in the treatment and/or prevention of rare diseases such as lymphangioleiomyomatosis.

## Patentansprüche

1. Verbindung der Formel (I) 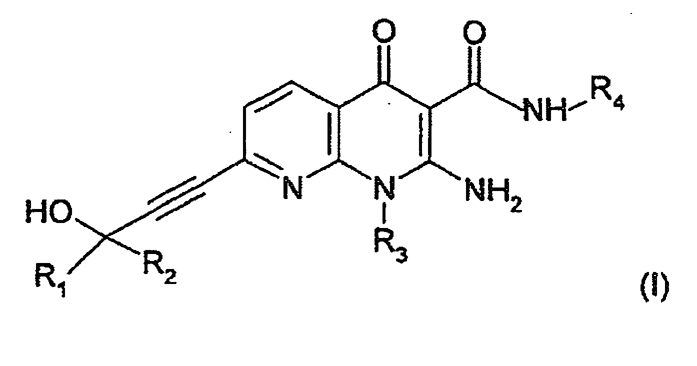 worin:
R₁ und R₂
(1) unabhängig voneinander für
• entweder ein Wasserstoffatom,
• oder eine C1-C7-Alkylgruppe, eine -CO-C1-C7-Alkylgruppe oder eine C3-C8-Cycloalkylgruppe, wobei die Alkyl- und Cycloalkylgruppen gegebenenfalls durch eine oder mehrere unter Halogenatomen und Hydroxy- und Alkoxygruppen ausgewählte Gruppen substituiert sind,
• oder eine Phenylgruppe, die gegebenenfalls durch eine oder mehrere unter Halogenatomen und C1-C4-Alkyl-, C1-C4-Alkoxy-, Hydroxy-, Halogenalkoxy-, Halogenalkyl-, -CN- und -NRR'-Gruppen ausgewählte Gruppen substituiert ist, wobei R und R' die unten angegebene Bedeutung besitzen,
• oder eine Heteroarylgruppe, die gegebenenfalls in beliebigen Positionen, einschließlich an einem Stickstoffatom des Heteroaryls, durch eine oder mehrere unter Halogenatomen und C1-C4-Alkyl- und -NRR'-Gruppen ausgewählte Gruppen substituiert ist, wobei R und R' die unten angegebene Bedeutung besitzen,
stehen,
(2) oder R₁ und R₂ zusammen mit dem Kohlenstoffatom, an das sie gebunden sind,
• entweder eine C4-C8-Cycloalkylgruppe
• oder eine 4- bis 8-gliedrige gesättigte heterocyclische Gruppe mit einem unter N-, O- und S-Atomen ausgewählten Heteroatom
bilden, wobei die heterocyclische Gruppe mit einer Phenylgruppe anelliert sein kann;
R₃ für:
• entweder eine lineare oder verzweigte C1-C7-Alkylgruppe oder eine C3-C7-Alkylgruppe, in der mindestens 3 Kohlenstoffatome cyclisiert sind, wobei die Alkylgruppe gegebenenfalls durch eine oder mehrere unter Halogenatomen und Hydroxy-, Alkoxy-, -NRR'-, Halogenalkyl- und -SO₂(C1C4)-Alkylgruppen ausgewählte Gruppen substituiert ist, wobei R und R' die unten angegebene Bedeutung besitzen,
• oder eine -(CH₂)ₙ-heterocyclische Gruppe, wobei n = 0, 1, 2 oder 3 und wobei die heterocyclische Gruppe zwischen 4 und 8 Glieder enthält und mindestens ein unter N-, O- und S-Atomen ausgewähltes Heteroatom enthält, wobei die heterocyclische Gruppe gegebenenfalls durch eine Oxogruppe substituiert ist,
• oder eine -(CH₂)ₙ-Heteroarylgruppe, wobei n = 0, 1, 2 oder 3 und wobei die Heteroarylgruppe 5 oder 6 Glieder enthält und ein oder mehrere unter Stickstoff, Sauerstoff und Schwefel ausgewählte Heteroatome enthält,
steht;
R₄ für ein Wasserstoffatom, eine C1-C4-Alkylgruppe oder eine C1-C4-Alkoxygruppe steht;
R und R' unabhängig voneinander für ein Wasserstoffatom oder eine C1-C4-Alkylgruppe stehen;
in Basen- oder Säureadditionssalzform sowie Enantiomere und Diastereoisomere davon sowie ein Gemisch davon.

2. Verbindung der Formel (I) nach Anspruch 1, **dadurch gekennzeichnet, daß**
R₁ und R₂
(1) unabhängig voneinander für
• entweder ein Wasserstoffatom,
• oder eine C1-C7-Alkylgruppe, eine -CO-C1-C7-Alkylgruppe oder eine C3-C8-Cycloalkylgruppe, wobei die Alkylgruppe gegebenenfalls durch eine oder mehrere unter Hydroxy- und Alkoxygruppen ausgewählte Gruppen substituiert ist,
• oder eine Phenylgruppe, die gegebenenfalls durch eine oder mehrere unter C1-C4-Alkoxygruppen ausgewählte Gruppen substituiert ist,
• oder eine Heteroarylgruppe
stehen,
(2) oder R₁ und R₂ zusammen mit dem Kohlenstoffatom, an das sie gebunden sind,
• entweder eine C4-C8-Cycloalkylgruppe
• oder eine 4- bis 8-gliedrige gesättigte heterocyclische Gruppe mit einem unter N-, O- und S-Atomen ausgewählten Heteroatom
bilden, wobei die heterocyclische Gruppe mit einer Phenylgruppe anelliert sein kann;
R₃ für:
• entweder eine lineare oder verzweigte C1-C7-Alkylgruppe oder eine C3-C7-Alkylgruppe, in der mindestens 3 Kohlenstoffatome cyclisiert sind, wobei die Alkylgruppe gegebenenfalls durch eine oder mehrere unter Hydroxy-, Alkoxy-, -NRR'-, Halogenalkyl- und -SO₂-(C1-C4)-Alkylgruppen ausgewählte Gruppen substituiert ist, wobei R und R' die unten angegebene Bedeutung besitzen,
• oder eine -(CH₂)ₙ-heterocyclische Gruppe, wobei n = 0, 1, 2 oder 3 und wobei die heterocyclische Gruppe zwischen 4 und 8 Glieder enthält und mindestens ein unter N- und O-Atomen ausgewähltes Heteroatom enthält, wobei die heterocyclische Gruppe gegebenenfalls durch eine Oxogruppe substituiert ist,
• oder eine -(CH₂)ₙ-Heteroarylgruppe, wobei n = 0 oder 1 und wobei die Heteroarylgruppe 5 oder 6 Glieder enthält und ein oder mehrere Stickstoff-Heteroatome enthält,
steht;
R₄ für eine C1-C4-Alkylgruppe oder eine C1-C4-Alkoxygruppe steht;
R und R' jeweils für eine lineare oder verzweigte C1-C4-Alkylgruppe stehen;
in Basen- oder Säureadditionssalzform sowie Enantiomere und Diastereoisomere davon sowie ein Gemisch davon.

3. Verbindung der Formel (I) nach Anspruch 1, **dadurch gekennzeichnet, daß**
R₁ und R₂
(1) unabhängig voneinander für
• entweder ein Wasserstoffatom,
• oder eine C1-C7-Alkylgruppe, eine -CO-C1-C7-Alkylgruppe oder eine C3-C8-Cycloalkylgruppe, wobei die Alkylgruppe gegebenenfalls durch eine oder mehrere unter Hydroxy- und Alkoxygruppen ausgewählte Gruppen substituiert ist,
• oder eine Phenylgruppe, die gegebenenfalls durch eine oder mehrere C1-C4-Alkoxygruppen substituiert ist,
• oder eine Heteroarylgruppe
stehen,
(2) oder R₁ und R₂ zusammen mit dem Kohlenstoffatom, an das sie gebunden sind,
• entweder eine C4-C8-Cycloalkylgruppe
• oder eine 4- bis 8-gliedrige gesättigte heterocyclische Gruppe mit einem Sauerstoff-Heteroatom bilden, wobei die heterocyclische Gruppe mit einer Phenylgruppe anelliert sein kann;
R₃ für:
• entweder eine lineare oder verzweigte C1-C7-Alkylgruppe oder eine C3-C7-Alkylgruppe, in der mindestens 3 Kohlenstoffatome cyclisiert sind, wobei die Alkylgruppe gegebenenfalls durch eine oder mehrere unter Hydroxy-, Alkoxy-, -NRR'-, Halogenalkyl- und -SO₂-(C1-C4)-Alkylgruppen ausgewählte Gruppen substituiert ist, wobei R und R' die unten angegebene Bedeutung besitzen,
• oder eine -(CH₂)ₙ-heterocyclische Gruppe, wobei n = 0, 1, 2 oder 3 und wobei die heterocyclische Gruppe zwischen 4 und 8 Glieder enthält und mindestens ein unter N- und O-Atomen ausgewähltes Heteroatom enthält, wobei die heterocyclische Gruppe gegebenenfalls durch eine Oxogruppe substituiert ist,
• oder eine -(CH₂)ₙ-Heteroarylgruppe, wobei n = 0 oder 1 und wobei die Heteroarylgruppe 5 oder 6 Glieder enthält und ein oder mehrere Stickstoff-Heteroatome enthält,
steht;
R₄ für eine C1-C4-Alkylgruppe oder eine C1-C4-Alkoxygruppe steht;
R und R' jeweils für eine lineare oder verzweigte C1-C4-Alkylgruppe stehen;
in Basen- oder Säureadditionssalzform sowie Enantiomere und Diastereoisomere davon sowie ein Gemisch davon.

4. Verbindung der Formel (I) nach Anspruch 1, **dadurch gekennzeichnet, daß**
R₁ und R₂
(1) unabhängig voneinander für
• entweder ein Wasserstoffatom,
• oder eine C1-C7-Alkylgruppe oder eine C3-C8-Cycloalkylgruppe, wobei die Alkylgruppe gegebenenfalls durch eine oder mehrere unter Hydroxy- und Alkoxygruppen ausgewählte Gruppen substituiert ist,
• oder eine Phenylgruppe, die gegebenenfalls durch eine oder mehrere C1-C4-Alkoxygruppen substituiert ist,
• oder eine Heteroarylgruppe
stehen,
(2) oder R₁ und R₂ zusammen mit dem Kohlenstoffatom, an das sie gebunden sind, eine C4-C8-Cycloalkylgruppe bilden;
R₃ für:
• entweder eine lineare oder verzweigte C1-C7-Alkylgruppe oder eine C3-C7-Alkylgruppe, in der mindestens 3 Kohlenstoffatome cyclisiert sind, wobei die Alkylgruppe gegebenenfalls durch eine oder mehrere unter Hydroxy-, Alkoxy-, Halogenalkyl- und -SO₂-(C1-C4)-Alkylgruppen ausgewählte Gruppen substituiert ist,
• oder eine -(CH₂)ₙ-heterocyclische Gruppe, wobei n = 0, 1, 2 oder 3 und wobei die heterocyclische Gruppe zwischen 4 und 8 Glieder enthält und mindestens ein unter N- und O-Atomen ausgewähltes Heteroatom enthält, wobei die heterocyclische Gruppe gegebenenfalls durch eine Oxogruppe substituiert ist,
• oder eine -(CH₂)ₙ-Heteroarylgruppe, wobei n = 0 oder 1 und wobei die Heteroarylgruppe 5 oder 6 Glieder enthält und ein oder mehrere Stickstoff-Heteroatome enthält,
steht;
R₄ für eine C1-C4-Alkylgruppe steht;
R und R' jeweils für eine lineare oder verzweigte C1-C4-Alkylgruppe stehen;
in Basen- oder Säureadditionssalzform sowie Enantiomere und Diastereoisomere davon sowie ein Gemisch davon.

5. Verbindung der Formel (I) nach Anspruch 1, **dadurch gekennzeichnet, daß** R₁ und R₂
**(1)** unabhängig voneinander für
• entweder ein Wasserstoffatom,
• oder eine C1-C7-Alkylgruppe, eine -CO-C1-C7-Alkylgruppe oder eine C3-C8-Cycloalkylgruppe, wobei die Alkylgruppe gegebenenfalls durch eine oder mehrere unter Hydroxy- und Alkoxygruppen ausgewählte Gruppen substituiert ist,
• oder eine Phenylgruppe, die gegebenenfalls durch eine oder mehrere C1-C4-Alkoxygruppen substituiert ist,
• oder eine Heteroarylgruppe
stehen,
(2) oder R₁ und R₂ zusammen mit dem Kohlenstoffatom, an das sie gebunden sind,
• entweder eine C4-C8-Cycloalkylgruppe
• oder eine 4- bis 8-gliedrige gesättigte heterocyclische Gruppe mit einem unter N-, O- und S-Atomen ausgewählten Heteroatom
bilden, wobei die heterocyclische Gruppe mit einer Phenylgruppe anelliert sein kann;
in Basen- oder Säureadditionssalzform sowie Enantiomere und Diastereoisomere davon sowie ein Gemisch davon.

6. Verbindung der Formel (I) nach Anspruch 1, **dadurch gekennzeichnet, daß** R₃ für:
• entweder eine lineare oder verzweigte C1-C7-Alkylgruppe oder eine C3-C7-Alkylgruppe, in der mindestens 3 Kohlenstoffatome cyclisiert sind, wobei die Alkylgruppe gegebenenfalls durch eine oder mehrere unter Hydroxy-, Alkoxy-, -NRR'- und Halogenalkylgruppen ausgewählte Gruppen substituiert ist, wobei R und R' die unten angegebene Bedeutung besitzen,
• oder eine -(CH₂)ₙ-heterocyclische Gruppe, wobei n = 0, 1, 2 oder 3 und wobei die heterocyclische Gruppe zwischen 4 und 8 Glieder enthält und mindestens ein unter N- und O-Atomen ausgewähltes Heteroatom enthält, wobei die heterocyclische Gruppe gegebenenfalls durch eine Oxogruppe substituiert ist,
• oder eine -(CH₂)ₙ-Heteroarylgruppe, wobei n = 0 oder 1 und wobei die Heteroarylgruppe 5 oder 6 Glieder enthält und ein oder mehrere Stickstoff-Heteroatome enthält,
steht;
in Basen- oder Säureadditionssalzform sowie Enantiomere und Diastereoisomere davon sowie ein Gemisch davon.

7. Verbindung der Formel (I) nach Anspruch 1, **dadurch gekennzeichnet, daß** R₄ für eine C1-C4-Alkylgruppe oder eine C1-C4-Alkoxygruppe steht;
in Basen- oder Säureadditionssalzform sowie Enantiomere und Diastereoisomere davon sowie ein Gemisch davon.

8. Verbindung der Formel (I) nach Anspruch 1, **dadurch gekennzeichnet, daß** R und R' jeweils für eine lineare oder verzweigte C1-C4-Alkylgruppe stehen, in Basen- oder Säureadditionssalzform sowie Enantiomere und Diastereoisomere davon sowie ein Gemisch davon.

9. Verbindung der Formel (I) nach Anspruch 1, **dadurch gekennzeichnet, daß** sie unter
■ 2-Amino-1-ethyl-7-(3-hydroxy-3-methylbut-1-in-1-yl)-N-methyl-4-oxo-1,4-dihydro-1,8-naphthyridin-3-carboxamid
■ (±)-2-Amino-1-ethyl-7-[(1-hydroxycyclopentyl)-ethinyl]-N-methyl-4-oxo-1,4-dihydro-1,8-naphthyridin-3-carboxamid
■ (±)-2-Amino-1-ethyl-7-(3-hydroxybut-1-in-1-yl)-N-methyl-4-oxo-1,4-dihydro-1,8-naphthyridin-3-carboxamid
■ (±)-2-Amino-1-ethyl-7-(3-hydroxy-3-methylpent-1-in-1-yl)-N-methyl-4-oxo-1,4-dihydro-1,8-naphthyridin-3-carboxamid
■ (±)-2-Amino-1-ethyl-7-(3-hydroxy-4-methoxy-3-methylbut-1-in-1-yl)-N-methyl-4-oxo-1,4-dihydro-1,8-naphthyridin-3-carboxamid
■ (±)-2-Amino-7-(3-cyclopentyl-3-hydroxyprop-1-in-1-yl)-1-ethyl-N-methyl-4-oxo-1,4-dihydro-1,8-naphthyridin-3-carboxamid
■ (±)-2-Amino-7-(3-cyclopropyl-3-hydroxyprop-1-in-1-yl)-1-ethyl-N-methyl-4-oxo-1,4-dihydro-1,8-naphthyridin-3-carboxamid
■ 2-Amino-1-ethyl-7-[(1-hydroxycyclobutyl)ethinyl]-N-methyl-4-oxo-1,4-dihydro-1,8-naphthyridin-3-carboxamid
■ (±)-2-Amino-7-(3,4-dihydroxy-3-methylbut-1-in-1-yl)-1-ethyl-N-methyl-4-oxo-1,4-dihydro-1,8-naphthyridin-3-carboxamid
■ 2-Amino-1-ethyl-7-[3-hydroxy-4-methoxy-3-(methoxymethyl)but-1-in-1-yl]-N-methyl-4-oxo-1,4-dihydro-1,8-naphthyridin-3-carboxamid
■ (±)-2-Amino-1-(cyclopropylmethyl)-7-(3-hydroxy-4-methoxy-3-methylbut-1-in-1-yl)-N-methyl-4-oxo-1,4-dihydro-1,8-naphthyridin-3-carboxamid
■ (±)-2-Amino-1-ethyl-7-(3-hydroxy-3-phenylbut-1-in-1-yl)-N-methyl-4-oxo-1,4-dihydro-1,8-naphthyridin-3-carboxamid
■ (±)-2-Amino-1-ethyl-7-[3-hydroxy-3-(3-thienyl)but-1-in-1-yl]-N-methyl-4-oxo-1,4-dihydro-1,8-naphthyridin-3-carboxamid
■ (±)-2-Amino-1-ethyl-7-[3-hydroxy-3-(3-methoxyphenyl)but-1-in-1-yl]-N-methyl-4-oxo-1,4-dihydro-1,8-naphthyridin-3-carboxamid
■ (±)-2-Amino-1-ethyl-7-[3-hydroxy-3-(4-methoxyphenyl)but-1-in-1-yl]-N-methyl-4-oxo-1,4-dihydro-1,8-naphthyridin-3-carboxamid
■ (±)-2-Amino-7-(3-hydroxy-4-methoxy-3-methylbut-1-in-1-yl)-1-(3-methoxypropyl)-N-methyl-4-oxo-1,4-dihydro-1,8-naphthyridin-3-carboxamid
■ (±)-2-Amino-1-cyclopentyl-7-(3-hydroxy-4-methoxy-3-methylbut-1-in-1-yl)-N-methyl-4-oxo-1,4-dihydro-1,8-naphthyridin-3-carboxamid
■ (±)-2-Amino-7-(3-hydroxy-4-methoxy-3-methylbut-1-in-1-yl)-1-isopropyl-N-methyl-4-oxo-1,4-dihydro-1,8-naphthyridin-3-carboxamid
■ (±)-2-Amino-7-(3-hydroxy-4-methoxy-3-methylbut-1-in-1-yl)-1-isobutyl-N-methyl-4-oxo-1,4-dihydro-1,8-naphthyridin-3-carboxamid
■ 2-Amino-7-(3-hydroxy-3-methylbut-1-in-1-yl)-1-(3-methoxypropyl)-N-methyl-4-oxo-1,4-dihydro-1,8-naphthyridin-3-carboxamid
■ (±)-2-Amino-1-ethyl-7-(3-hydroxy-4-methoxy-3-phenylbut-1-in-1-yl)-N-methyl-4-oxo-1,4-dihydro-1,8-naphthyridin-3-carboxamid
■ (±)-2-Amino-1-(tetrahydropyran-4-yl)-7-(3-hydroxy-4-methoxy-3-methylbut-1-in-1-yl)-N-methyl-4-oxo-1,4-dihydro-1,8-naphthyridin-3-carboxamid
■ (±)-2-Amino-1-cyclohexyl-7-(3-hydroxy-4-methoxy-3-methylbut-1-in-1-yl)-N-methyl-4-oxo-1,4-dihydro-1,8-naphthyridin-3-carboxamid
■ (±)-2-Amino-1-ethyl-7-[3-hydroxy-3-(1,3-thiazol-2-yl)but-1-in-1-yl]-N-methyl-4-oxo-1,4-dihydro-1,8-naphthyridin-3-carboxamid
■ (±)-2-Amino-1-ethyl-7-[3-hydroxy-3-(methoxymethyl)pent-1-in-1-yl]-N-methyl-4-oxo-1,4-dihydro-1,8-naphthyridin-3-carboxamid
■ (±)-2-Amino-7-(3-hydroxy-4-methoxy-3-methylbut-1-in-1-yl)-N-methyl-4-oxo-1-[3-(2-oxopyrrolidin-1-yl)propyl]-1,4-dihydro-1,8-naphthyridin-3-carboxamid
■ (±)-2-Amino-7-(3-hydroxy-4-methoxy-3-methylbut-1-in-1-yl)-N-methyl-4-oxo-1-propyl-1,4-dihydro-1,8-naphthyridin-3-carboxamid
■ (±)-2-Amino-7-(3-hydroxy-4-methoxy-3-methylbut-1-in-1-yl)-N-methyl-4-oxo-1-(2,2,2-trifluorethyl)-1,4-dihydro-1,8-naphthyridin-3-carboxamid
■ (±)-2-Amino-7-(3-hydroxy-3-methylbut-1-in-1-yl)-N-methyl-4-oxo-1-(tetrahydrofuran-2-ylmethyl)-1,4-dihydro-1,8-naphthyridin-3-carboxamid
■ (±)-2-Amino-7-(3-hydroxy-4-methoxy-3-methylbut-1-in-1-yl)-N-methyl-4-oxo-1-(pyridin-2-ylmethyl)-1,4-dihydro-1,8-naphthyridin-3-carboxamid
■ (±)-2-Amino-1-ethyl-7-(3-hydroxy-3-pyrazin-2-ylbut-1-in-1-yl)-N-methyl-4-oxo-1,4-dihydro-1,8-naphthyridin-3-carboxamid
■ (±)-2-Amino-7-(3-cyclopropyl-3-hydroxy-4-methoxybut-1-in-1-yl)-1-ethyl-N-methyl-4-oxo-1,4-dihydro-1,8-naphthyridin-3-carboxamid
■ (+)-2-Amino-1-ethyl-7-[3-hydroxy-3-(2-thienyl)but-1-in-1-yl]-N-methyl-4-oxo-1,4-dihydro-1,8-naphthyridin-3-carboxamid
■ (+)-2-Amino-1-ethyl-7-[3-hydroxy-3-(2-methoxyphenyl)but-1-in-1-yl]-N-methyl-4-oxo-1,4-dihydro-1,8-naphthyridin-3-carboxamid
■ (±)-2-Amino-1-cyclopentyl-7-(3,4-dihydroxy-3-methylbut-1-in-1-yl)-N-methyl-4-oxo-1,4-dihydro-1,8-naphthyridin-3-carboxamid
■ (±)-2-Amino-7-(4-ethoxy-3-hydroxy-3-methylbut-1-in-1-yl)-1-ethyl-N-methyl-4-oxo-1,4-dihydro-1,8-naphthyridin-3-carboxamid
■ (±)-2-Amino-1-(trans-4-hydroxycyclohexyl)-7-(3-hydroxy-4-methoxy-3-methylbut-1-in-1-yl)-N-methyl-4-oxo-1,4-dihydro-1,8-naphthyridin-3-carboxamid
■ (±)-2-Amino-7-(3-hydroxy-4-methoxy-3-methylbut-1-in-1-yl)-N-methyl-1-[3-(methylsulfonyl)propyl]-4-oxo-1,4-dihydro-1,8-naphthyridin-3-carboxamid
■ (-)-2-Amino-1-(cyclopropylmethyl)-7-(3-hydroxy-4-methoxy-3-methylbut-1-in-1-yl)-N-methyl-4-oxo-1,4-dihydro-1,8-naphthyridin-3-carboxamid
■ (+)-2-Amino-1-(cyclopropylmethyl)-7-(3-hydroxy-4-methoxy-3-methylbut-1-in-1-yl)-N-methyl-4-oxo-1,4-dihydro-1,8-naphthyridin-3-carboxamid
■ (+)-2-Amino-7-(3,4-dihydroxy-3-methylbut-1-in-1-yl)-1-ethyl-N-methyl-4-oxo-1,4-dihydro-1,8-naphthyridin-3-carboxamid
■ (-)-2-Amino-7-(3,4-dihydroxy-3-methylbut-1-in-1-yl)-1-ethyl-N-methyl-4-oxo-1,4-dihydro-1,8-naphthyridin-3-carboxamid
■ (-)-2-Amino-1-ethyl-7-(3-hydroxy-3-phenylbut-1-in-1-yl)-N-methyl-4-oxo-1,4-dihydro-1,8-naphthyridin-3-carboxamid
■ (+)-2-Amino-1-ethyl-7-(3-hydroxy-3-phenylbut-1-in-1-yl)-N-methyl-4-oxo-1,4-dihydro-1,8-naphthyridin-3-carboxamid
■ (-)-2-Amino-7-(3-hydroxy-4-methoxy-3-methylbut-1-in-1-yl)-1-(3-methoxypropyl)-N-methyl-4-oxo-1,4-dihydro-1,8-naphthyridin-3-carboxamid
■ (+)-2-Amino-7-(3-hydroxy-4-methoxy-3-methylbut-1-in-1-yl)-1-(3-methoxypropyl)-N-methyl-4-oxo-1,4-dihydro-1,8-naphthyridin-3-carboxamid
■ (-)-2-Amino-1-cyclopentyl-7-(3-hydroxy-4-methoxy-3-methylbut-1-in-1-yl)-N-methyl-4-oxo-1,4-dihydro-1,8-naphthyridin-3-carboxamid
■ (+)-2-Amino-1-cyclopentyl-7-(3-hydroxy-4-methoxy-3-methylbut-1-in-1-yl)-N-methyl-4-oxo-1,4-dihydro-1,8-naphthyridin-3-carboxamid
■ (-)-2-Amino-7-(3-hydroxy-4-methoxy-3-methylbut-1-in-1-yl)-1-isopropyl-N-methyl-4-oxo-1,4-dihydro-1,8-naphthyridin-3-carboxamid
■ (+)-2-Amino-7-(3-hydroxy-4-methoxy-3-methylbut-1-in-1-yl)-1-isopropyl-N-methyl-4-oxo-1,4-dihydro-1,8-naphthyridin-3-carboxamid
■ (-)-2-Amino-1-cyclohexyl-7-(3-hydroxy-4-methoxy-3-methylbut-1-in-1-yl)-N-methyl-4-oxo-1,4-dihydro-1,8-naphthyridin-3-carboxamid
■ (+)-2-Amino-1-cyclohexyl-7-(3-hydroxy-4-methoxy-3-methylbut-1-in-1-yl)-N-methyl-4-oxo-1,4-dihydro-1,8-naphthyridin-3-carboxamid
■ (-)-2-Amino-1-ethyl-7-(3-hydroxy-4-methoxy-3-methylbut-1-in-1-yl)-N-methyl-4-oxo-1,4-dihydro-1,8-naphthyridin-3-carboxamid
■ (+)-2-Amino-1-ethyl-7-(3-hydroxy-4-methoxy-3-methylbut-1-in-1-yl)-N-methyl-4-oxo-1,4-dihydro-1,8-naphthyridin-3-carboxamid
■ (-)-2-Amino-7-(3-hydroxy-4-methoxy-3-methylbut-1-in-1-yl)-N-methyl-4-oxo-1-propyl-1,4-dihydro-1,8-naphthyridin-3-carboxamid
■ (+)-2-Amino-7-(3-hydroxy-4-methoxy-3-methylbut-1-in-1-yl)-N-methyl-4-oxo-1-propyl-1,4-dihydro-1,8-naphthyridin-3-carboxamid
■ (-)-2-Amino-7-(3-hydroxy-4-methoxy-3-methylbut-1-in-1-yl)-N-methyl-4-oxo-1-(2,2,2-trifluorethyl)-1,4-dihydro-1,8-naphthyridin-3-carboxamid
■ (+)-2-Amino-7-(3-hydroxy-4-methoxy-3-methylbut-1-in-1-yl)-N-methyl-4-oxo-1-(2,2,2-trifluorethyl)-1,4-dihydro-1,8-naphthyridin-3-carboxamid
■ (+)-2-Amino-1-ethyl-7-[3-hydroxy-3-(methoxymethyl)pent-1-in-1-yl]-N-methyl-4-oxo-1,4-dihydro-1,8-naphthyridin-3-carboxamid
■ (-)-2-Amino-1-ethyl-7-[3-hydroxy-3-(methoxymethyl)pent-1-in-1-yl]-N-methyl-4-oxo-1,4-dihydro-1,8-naphthyridin-3-carboxamid
■ (+)-2-Amino-1-ethyl-7-[3-hydroxy-3-(3-thienyl)but-1-in-1-yl]-N-methyl-4-oxo-1,4-dihydro-1,8-naphthyridin-3-carboxamid
■ (-)-2-Amino-1-ethyl-7-[3-hydroxy-3-(3-thienyl)but-1-in-1-yl]-N-methyl-4-oxo-1,4-dihydro-1,8-naphthyridin-3-carboxamid ausgewählt ist.

10. Verbindung der Formel (VII) 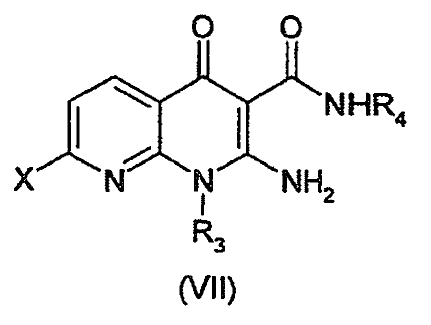 worin X für ein Halogenatom steht und R₃ und R₄ die in einem der vorhergehenden Ansprüche angegebene Bedeutung besitzen.

11. Verfahren zur Herstellung einer Verbindung der Formel (I) nach einem der Ansprüche 1 bis 9, **dadurch gekennzeichnet, daß** man eine Verbindung der Formel (VII) 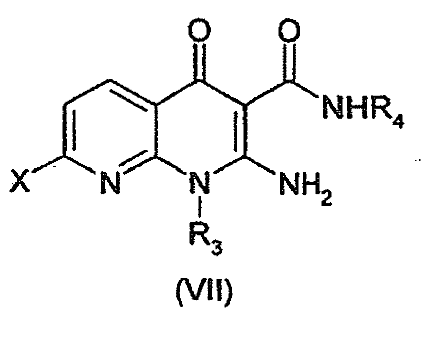 worin X für ein Halogenatom steht und R₃ und R₄ die in einem der vorhergehenden Ansprüche angegebene Bedeutung besitzen,
mit einer Verbindung der Formel (VIII) 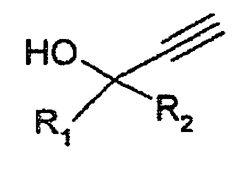 worin R₁ und R₂ die in einem der vorhergehenden Ansprüche angegebene Bedeutung besitzen,
umsetzt.

12. Medikament, **dadurch gekennzeichnet, daß** es eine Verbindung der Formel (I) nach einem der Ansprüche 1 bis 9 oder ein Additionssalz dieser Verbindung mit einer pharmazeutisch unbedenklichen Säure oder auch ein Enantiomer oder ein Diastereoisomer der Verbindung der Formel (I) oder ein Gemisch davon umfaßt.

13. Pharmazeutische Zusammensetzung, **dadurch gekennzeichnet, daß** sie eine Verbindung der Formel (I) nach einem der Ansprüche 1 bis 9 oder ein pharmazeutisch unbedenkliches Salz oder ein Enantiomer oder ein Diastereoisomer dieser Verbindung oder ein Gemisch davon sowie mindestens einen pharmazeutisch unbedenklichen Hilfsstoff umfaßt.

14. Kombination von mindestens einer Verbindung der Formel (I) nach einem der Ansprüche 1 bis 9 mit mindestens einem Chemotherapiemittel, das unter:
• Alkylierungsmitteln,
• Interkalationsmitteln,
• Antimikrotubulusmitteln,
• Antimitotika,
• Antimetaboliten,
• Antiproliferationsmitteln,
• Antibiotika,
• Immunmodulatoren,
• Antiphlogistika,
• Kinaseinhibitoren,
• antiangiogenen Mitteln,
• antivaskulären Mitteln und
• östrogenen und androgenen Hormonen ausgewählt ist.

15. Verbindung der Formel (I) nach einem der Ansprüche 1 bis 9 zur Verwendung zur Behandlung und/oder Prävention von Krebserkrankungen und Metastasen davon.

16. Verbindung der Formel (I) nach Anspruch 15 zur Verwendung zur Behandlung und/oder Prävention von Glioblastomen, multiplen Myelomen, myelodysplasischen Syndromen, Kaposi-Sarkomen, kutanen Angiosarkomen, soliden Tumoren, Lymphomen, Melanomen, Brustkrebserkrankungen, Kolorektalkrebserkrankungen, Lungenkrebserkrankungen einschließlich nichtkleinzelligen Krebserkrankungen, Bauchspeicheldrüsenkrebserkrankungen, Prostatakrebserkrankungen, Nierenkrebserkrankungen, Kopf- und Halskrebserkrankungen, Leberkrebserkrankungen, Eierstockkrebserkrankungen, Krebserkrankungen der Atemwege und des Thorax und anderen Tumoren, die VEGFR-3 exprimieren oder an denen ein Angiogenese- oder Lymphangiogeneseprozeß beteiligt ist.

17. Verbindung der Formel (I) zur Verwendung zur Herstellung eines Medikaments zur Behandlung und/oder Prävention von Erkrankungen, die unter Arthrose, Restenose, Psoriasis, Hämangiomen, Lymphangiomen, Glaukomen, Glomerulonephritiden, diabetischen Nephropathien, Nephrosklerosis, thrombotischen mikroangiopathischen Syndromen, Leberzirrhose, Atherosklerose, Organtransplantatabstoßung und Augenerkrankungen, an denen ein Angiogenese- oder Lymphangiogeneseprozeß beteiligt ist, ausgewählt sind.

18. Verbindung der Formel (I) nach einem der Ansprüche 1 bis 9 zur Herstellung eines Medikaments zur Behandlung und/oder Prävention von chronischer oder nichtchronischer Entzündung, Infektion mit Mikroorganismen und Autoimmunerkrankungen, wie rheumatoider Arthritis.

19. Verbindung der Formel (I) nach einem der Ansprüche 1 bis 9 zur Verwendung zur Behandlung und/oder Prävention von seltenen Erkrankungen wie Lymphangioleiomyomatose.
